(19) Europäisches Patentamt
European Patent Office
Office européen des brevets

(11) EP 0 448 697 B1

(12) **FASCICULE DE BREVET EUROPEEN**

(45) Date de publication et mention de la délivrance du brevet:
**10.04.1996 Bulletin 1996/15**

(21) Numéro de dépôt: **90916133.3**

(22) Date de dépôt: **15.10.1990**

(51) Int Cl.[6]: **C07C 403/06**, C07C 403/18

(86) Numéro de dépôt international:
**PCT/FR90/00742**

(87) Numéro de publication internationale:
**WO 91/05754 (02.05.1991 Gazette 1991/10)**

(54) **ANALOGUES DE LA VITAMINE A, LEURS APPLICATIONS, NOTAMMENT EN TANT QUE MOLECULES CYTOTROPHIQUES ET CYTOPROTECTRICES, ET COMPOSITIONS PHARMACEUTIQUES LES CONTENANT**

ANALOGE VON VITAMIN A UND IHRE VERWENDUNG, INSBESONDERE ALS CYTOTROPHISCHE UND CYTOPROTECTIVE SUBSTANZEN SOWIE DIESE SUBSTANZEN ENTHALTENDE ARZNEIMITTEL

VITAMIN A ANALOGUES AND THEIR APPLICATIONS, IN PARTICULAR AS CYTOTROPHIC AND CYTOPROTECTIVE MOLECULES, AND PHARMACEUTICAL COMPOSITIONS CONTAINING SAME

(84) Etats contractants désignés:
**AT BE CH DE DK ES FR GB GR IT LI LU NL SE**

(30) Priorité: **13.10.1989 FR 8913456**
**14.02.1990 FR 9001771**

(43) Date de publication de la demande:
**02.10.1991 Bulletin 1991/40**

(73) Titulaire: **NEUROFIT**
**F-67000 Strasbourg (FR)**

(72) Inventeur: **BORG, Jacques**
**F-67800 Bischheim (FR)**

(74) Mandataire: **Gutmann, Ernest et al**
**Ernest Gutmann - Yves Plasseraud S.A.**
**3, rue Chauveau-Lagarde**
**F-75008 Paris (FR)**

(56) Documents cités:

| | |
|---|---|
| **EP-A- 78 533** | **WO-A-84/01899** |
| **WO-A-90/04388** | **DE-A- 2 551 914** |
| **DE-B- 2 459 985** | **US-A- 3 646 215** |
| **US-A- 3 689 667** | **US-A- 4 874 794** |

- **Chemical Abstracts, volume 101, no. 4, 23 juillet 1984 (Columbus, Ohio, US) P. K. Das et al.: "Twenty-two carbon homologue of 11-cis-retinal. Photophysical and photochemical properties", voir page 534, abrégé 38706b, & Photochem. Photobiol. 1984, 39(3), 313-18**
- **Chemical Abstracts, volume 94, no. 21, 25 mai 1981 (Columbus, Ohio, US) P.K. Das et al.: "Spectroscopy of polyenes. IV. Absorption and emission spectral properties of polyene alcohols related to retinol as homologues", voir page 753, abrégé 175328f, & Photochem. Photobiol. 1980, 32(6), 739-48**
- **Chemical Abstracts, volume 93, no. 7, 18 août 1980 (Columbus, Ohio, US) D.D. Auperin et al.: "Characterization of virucidal activities of retinoids", voir page 44, abrégé 61142q, & Curr. Chemother. Infect. Dis., Proc. Int. Congr. Chemother. 11th 1979 (Pub. 1980) 2, 1368-70**

EP 0 448 697 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

- Chemical Abstracts, volume 75, no. 9, 30 août 1971 (Columbus, Ohio, US) H.P. Ehrlich: "Effects of beta-carotene, vitamin A and glucocorticoids on collagen synthesis in wounds", voir page 230; colonne de droite; abrégé no. 61125q & Proc. Soc. Exp. Biol. Med., 1971, 137(3), 936-8
- Ehrlich et al.: "Effects of beta-carotene, vitamin A, and glucocorticoids on collagen synthesis in wounds", voir page 230, abrégé 61125q, & Proc. Soc. Exp. Biol. Med. 1971, 137(3), 936-8
- Chemical Abstracts, volume 100, no. 19, 7 mai 1984, (Columbus, Ohio, US) G. Mozsik et al.: "Cytoprotective effect of vitamin A and carotenoids on the injury of the gastric mucosa caused by 0.6 M hydrochloric acid", voir page 449, abrégé 155572v, & Tapl. Hel. Fel. Magy 8th 1981, (Pub. 1983) 781-91
- Journal of Cellular Physiology, 124(1) (1985), pp. 120-124.
- Archives of Biochemistry and Biophysics, 238(1) (1985), pp. 1-9
- Patent Abstracts of Japan, volume 8, no. 178(C-238)(1615), 16 août 1984, & JP, A, 5973513 (EISAI), 25 avril 1984, voir le document en entier

## Description

La présente invention concerne des dérivés des alcools gras à longue chaîne hydrocarbonée, ainsi que des compositions pharmaceutiques comprenant ces dérivés, et l'utilisation de ces dernières, notamment en tant que médicaments cytotrophiques et cytoprotecteurs.

Il est généralement admis que le tissu nerveux des mammifères adultes ne se régénère pas à la suite d'une lésion. Cette absence de régénérescence peut être due soit à un défaut de signaux capables d'induire la régénérescence, soit à une lésion irréversible qui a conduit à la mort neuronale (A.J. AGUAYO, in : A. BIGNAMI, F.E. BLOOM, C.L. BOLIS et A. ADELOYE eds, CNS Plasticity and Repair, Raven Press (N.Y.) (1985) pp 31-40). Cependant, on peut éviter la mort neuronale en protégeant le tissu nerveux des conséquences de l'ischémie ou en lui fournissant un micro-environnement favorable, en particulier des composés neurotrophiques ou neuroprotecteurs ; ces composés pourraient également favoriser la régénérescence du tissu nerveux s'ils sont administrés avant que la mort ne soit intervenue.

Les facteurs neurotrophiques sont des agents capables de favoriser la survie des neurones et de stimuler la différenciation neuronale (J.R. PEREZ-POLO, in J.E. BOTTENSTEIN et G. SATO eds, Cell Culture in the neurosciences, Plenum Publishing Corp. (1985) pp 95-123). L'activation du métabolisme cellulaire peut avoir pour conséquence la formation d'un réseau de neurites orienté vers la cible à innerver. La mise en évidence de facteurs neurotrophiques peut se faire in vitro à l'aide de cultures de cellules nerveuses.(G. BARBIN, I. SELAK, M. MANTHORPE, et S. VARON ; NEUROSCIENCE 12 : 33-43 (1984)). Des cultures de cellules dissociées de diverses régions du système nerveux central (SNC) ont permis de montrer l'effet de ces facteurs neurotrophiques sur la survie des neurones et sur leur maturation.

Par ailleurs, la lésion expérimentale du septum médian chez le rat entraîne une perte importante de neurones cholinergiques (D. M. ARMSTRONG et al, COMP. NEUROL. 264 : 421-436 (1987) ; F.H. GAGE et al, BRAIN RES. 268 : 27-37 (1983)). Certaines maladies, comme la maladie d'Alzheimer ou de Parkinson, la chorée de Huntington ou la sclérose latérale amyotrophique sont la conséquence de la disparition progressive de certains neurones (J.T. COYLE et al, SCIENCE, 219 : 1184-1190 (1983) ; J.B. MARTIN, NEUROLOGY 34 : 1059-1072 (1984) ; M.D. YAHR et K.J. BREGMANN, NEUROLOGY vol., Raven Press (N.Y.) (1985)). Certaines études ont suggéré que cette perte de neurones pouvait être évitée par l'administration par voie intracérébroventriculaire de facteurs neurotrophiques comme le NGF (Nerve Growth Factor) ou le FGF (Fibroblast Growth Factor) (L.F. KROMER, Science 235 : 214-216 (1987) ; K.J. ANDERSON et al, Nature 332-360-361 (1988) ; T. HAGG et al, Exp. Neurol. 101 : 303-312 (1988)).

La mort neuronale pourrait également être liée à la présence de composés neurotoxiques. Certains acides aminés excitateurs ont une action excitotoxique sur les neurones ; quand on les injecte dans le cerveau de rats, ils provoquent un dégénérescence de certaines zones (J.T. COYLE et R. SCHWARCZ, in : Handbook of Chemical Neuroanatomy, Elsevier, Amsterdam (1983) pp 508-527). Ce phénomène est proche de celui de la dégénérescence neuronale observée dans la zone de l'hippocampe chez l'homme et qui est appelée sclérose de la corne d'Ammon (Meldrum, Clin. Sci. 68 : 112-113 (1985)). On peut reproduire ce type de lésion par des injections intracérébrales de composés excitotoxiques, comme l'acide iboténique. Il a été suggéré que la perte de neurones hippocampiques qu'on observe dans la maladie d'Alzheimer, et à la suite d'une ischémie cérébrale, pourrait être liée à la forte densité de récepteurs au glutamate présente sur ces neurones (S.M. ROTHMANN et J.W. OLNEY, Ann. Neurol. ; 19 : 105-111 (1986)). Les acides aminés neurotoxiques peuvent aussi induire des lésions à distance par l'intermédiaire des projections glutamatergiques et qui ressemblent aux lésions observées dans l'épilepsie (J.V. NADLER et al, Nature 271 : 676-677 (1978) et la chorée de Huntington (J.T. COYLE et R. SCHWARZCZ, Nature 263 : 244-246 (1976)). Les excitotoxines pourraient aussi intervenir dans la génèse des lésions ischémiques ; l'ischémie augmente la concentration extra-cellulaire en acides aminés excitateurs provoquant une hyperstimulation, un gonflement neuronal, puis une lyse cellulaire (Meldrum, Adv. Neurol.; 44 : 849-855 (1986) ; S.M. ROTHMANN et J.W. OLNEY, Ann. Neurol. 19 : 105-111 (1986)). De même au cours de l'épilepsie une libération accrue de neurotransmetteur pourrait produire des lésions neurodégénératives du même type (Y. BENARI, Neuroscience 14 : 375-403 (1985)).

Les inventeurs ont démontré que l'alcool gras à longue chaîne, le n-hexacosanol, possède des activités neurotrophiques et neuroprotectrices in vitro. Cet alcool gras à longue chaîne a été isolé de la plante tropicale, Hygrophila erecta, Hochr. (Acanthacées) (Borg et al, Neuroscience Lett. 213 : 406-410 (1987)). Si on l'ajoute à une concentration de 500 nM à des neurones de rat foetaux en culture, il augmente la croissance neuritique par un facteur de 4 à 6 ainsi que le nombre de collatérales, en particulier pour les neurones multipolaires. Ce composé augmente également de façon importante la différenciation biochimique des neurones en culture : il augmente le taux de protéines et double les activités spécifiques de deux enzymes neuronaux, la glutaminase activée par le phosphate et l'énolase neurospécifique, de 32 et 78 % respectivement.

Cependant, les résultats de l'étude in vitro des propriétés du n-hexacosanol ne permettent pas de juger de son éventuelle activité in vivo, compte tenu des différences existant entre les conditions d'environnement in vitro et in vivo.

Par ailleurs, le n-hexacosanol administré par voie périphérique atténue de façon significative dans le cerveau la dégénéresence neuronale après axotomie. Il réduit également de façon importante la perte de neurones consécutive

à une injection de neurotoxines dans le cerveau. Cet effet confirme les propriétés neurotrophiques et neuroprotectrices du n-hexacosanol.

L'invention a pour objet des dérivés d'alcools gras à longue chaîne, issus de composés terpéniques, présentant de puissantes propriétés neurotrophiques et neuroprotectrices in vitro et in vivo sur divers tissus nerveux.

Ces dérivés sont capables de traverser la barrière hématoencéphalique et ont une biodisponibilité bien supérieure aux autres facteurs neurotrophiques actuellement connus.

De manière avantageuse, les dérivés de l'invention atteignent des cibles cellulaires spécifiques, ce qui en fait des outils thérapeutiques de première importance.

L'invention a également pour objet des dérivés d'alcools gras à longue chaîne présentant des propriétés cytotrophiques et cytoprotectrices à l'égard d'une grande diversité de cellules de l'organisme.

L'invention concerne des dérivés d'alcools gras répondant à la formule suivante :

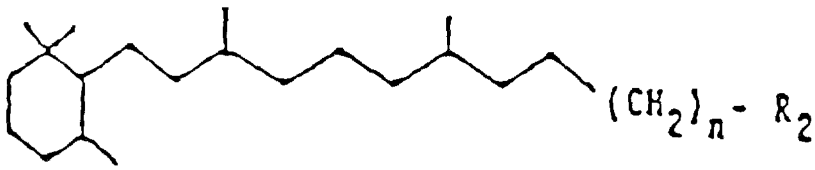

comportant, le cas échéant, 1 à 10, et de préférence 1 à 5, double(s) liaison(s) et dans laquelle

$R_2$ représente :

(1) CN;

(2) OR, R représentant un atome d'hydrogène, ou un groupe alcoyle de 1 à 3 atomes de carbone ou un groupe

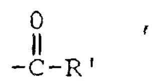

ou

(3)

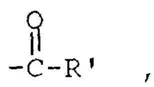

R' représentant, aux points (2) et (3) ci-dessus :

(a) un atome d'hydrogène,

(b) un groupe alcoyle de 1 à 5 atomes de carbone, de préférence de 1 à 3 atomes de carbone,

(c) un groupe alcoyle de 1 à 3 atomes de carbone,

(d) un groupe de formule $OR_b$, $R_b$ représentant un groupe alcoyle de 1 à 3 atomes de carbone, ou

(e) un groupe amino de formule $-NR'_1R'_2$, dans laquelle $R'_1$ et $R'_2$ identiques ou différents représentent H ou un groupe alcoyle de 1 à 3 atomes de carbone et n est un nombre entier variant de 1 à 10. Et, plus particulièrement les analogues saturés et insaturés de la vitamine A (ou rétinal) répondant à la formule suivante :

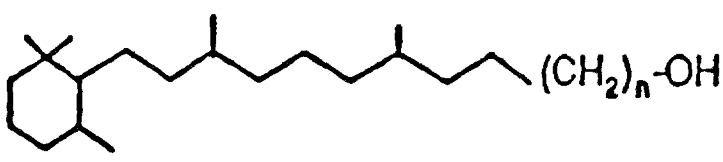

comportant, le cas échéant, 1 à 10, et de préférence 1 à 5 double(s) liaison(s), et dans laquelle n est un nombre entier variant de 1 à 10. Les poids moléculaires des analogues saturés et insaturés de la vitamine A de l'invention sont de l'ordre de 280 à 447.

Les dérivés saturés et insaturés de la vitamine A selon l'invention sont obtenus, notamment en couplant, par la

réaction de Wittig, le rétinal de formule :

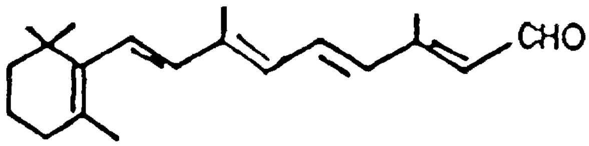

avec un ylure de dérivé de bromure de tetrahydropyranne (bromure-THP) de formule :

$$\Phi_3P{=}CH(CH_2)_{n-1}\text{-}OTHP$$
$$H^+$$

dans laquelle n est un nombre entier variant de 1 à 10, ce dernier composé étant lui-même obtenu par action d'un sel de bromure de tetrahydropyranne de formule : Br-$CH_2$-$(CH_2)_{n-1}$-OTHP (THP = tetrahydropyranne) sur un triphenylphosphine de formule :

$$\Phi_3P \; (\Phi = C_6H_5)$$

L'ylure alors obtenu est mis en contact avec le rétinal en présence de butyllithium, ce qui conduit à l'obtention d'un dérivé de formule :

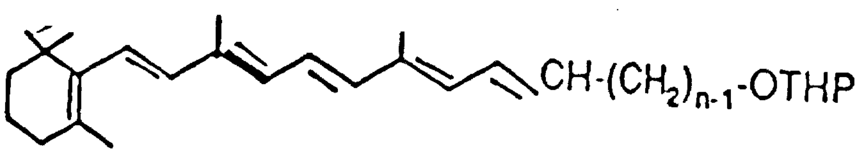

Ce dernier est ensuite hydrogéné pendant une durée déterminée ce qui conduit au dérivé de formule

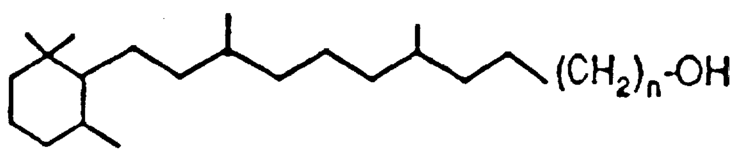

dans lequel n = 1 à 10, totalement saturé ou comportant 1 à 10 double(s) liaison(s) en fonction du temps d'hydrogénation.

Les dérivés d'alcools gras de l'invention précédemment décrits étant peu solubles en milieu aqueux, leur emploi pour des études biologiques ou pour des applications thérapeutiques peuvent présenter des difficultés méthodologiques. Pour remédier à ces inconvénients, des prodrogues hydrosolubles représentées par les esters de l'acide monophosphorique ont été synthétisés. Ces composés sont hydrolysés dans l'organisme pour donner les produits de départ à savoir les dérivés de l'invention pour lesquels R = H.

Ces composés sont particulièrement hydrosolubles (plus de 10 % dans l'eau selon les cas), donc les solutions obtenues sont compatibles avec des injections intraveineuses. Ils améliorent la biodisponibilité des dérivés de l'invention, ce qui permet de potentialiser les effets biologiques observés. Ainsi la présence du groupe phosphate a permis d'administrer une solution aqueuse de corticostéroides sans perte de l'activité biologique (R.J. W. CREMLYN et I. Khattak, Chemistry of steroid phosphates. Phosphorus 27 (1976) 237-246).

Le radical représenté par $R_3$ permet un meilleur ciblage du dérivé de l'invention. Il peut favoriser la pénétration du dérivé à travers des membranes (membrane bactérienne) ou la diffusion à partir des capillaires du tractus digestif, de la membrane méningée ou d'autres tissus (F. IGLESIAS-GUERRA, J.M. NEUMANN et T. HUYNH-DINH, Synthetic 6-glutamatecosyl phospholipid as a drug transport system/Tetrahedron Lett. 28 (1987) 3581-3584). Ce radical permet également au dérivé de se fixer sur un récepteur spécifique ; le dérivé agira donc spécifiquement sur les cellules qui présentent un tel récepteur. Un raisonnement similaire a conduit à la synthèse de dérivés phosphatés de certaines hormones stéroides (L. LEBEAU , C. MIOSKOWSXI et P. OUDET, Synthesis of phospholipids linked to steroid hormone derivatives. Chemistry and Physics of Lipids, 46 (1988) 57-62).

Les dérivés hydrosolubles de l'invention pour lesquels R représente le groupe

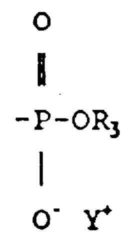

dans lequel $R_3$ et $y^+$ sont tels que définis ci-dessus, sont obtenus par phosphorylation d'un dérivé monomère ou dimère de l'invention, pour lequel R = H, avec l'ophenylènephosphochloridate, ou avec le bisphosphochloridate suivie d'une hydrolyse oxydative, notamment par le tétracétate de plomb.

Les conditions opératoires de l'ensemble des réactions décrites ci-dessus seront plus particulièrement développées dans la description détaillée qui suit :

Les dérivés de l'invention favorisent la régénérescence nerveuse et ont un effet neuroprotecteur; ils présentent notamment les propriétés suivantes.

Ils augmentent l'extension de neurites in vitro et in vivo, et facilitent la réparation à la suite de lésions traumatiques du SNC et du SNP (système nerveux périphérique), en favorisant l'extension de neurites et la formation de nouveaux contacts cellulaires et donc la récupération fonctionnelle. Ils atténuent l'effet de l'ischémie, favorisent la survie neuronale et réduisent la dégénérescence wallérienne après une lésion.

Ils protègent les neurones de la mort cellulaire consécutive à la présence de composés neurotoxiques, comme des métaux lourds ou des composés neuroexcitateurs, comme le glutamate et ses analogues.

Les dérivés de l'invention sont des facteurs cytotrophiques, notamment, pour les cellules de la peau et les cellules endothéliales. Ils favorisent la réparation des lésions de certaines couches cellulaires comme les cellules de la peau ou les cellules de l'endothélium vasculaire. Ces dérivés augmentent la différenciation des cellules du derme, de l'épiderme ou les cellules endothéliales ; ils augmentent la formation de prolongements et le comblement de la lésion par les cellules qui la bordent.

Les dérivés de l'invention ont un effet cytoprotecteur qui s'exerce, notamment, sur les cellules hépatiques, les cellules musculaires striées cardiaques et les cellules glomérulaires rénales. Ils protègent ces cellules contre des lésions chimiques ou liées à une ischémie. Ces dérivés permettent également de protéger le tissu cardiaque contre les conséquences d'une interruption du flux sanguin, en particulier dans les artères coronaires. Les dérivés de l'invention permettent donc de protéger les cellules hépatiques, rénales et musculaires cardiaques contre leur dégénérescence d'origine chimique ou ischémique.

Les dérivés de l'invention présentent également en effet trophique sur les cellules du glomérule rénal. Ils permettent une meilleure synthèse et la sécrétion de facteurs fibrinolytiques, comme les activeurs du plasminogène. Ils favorisent donc la fibrinolyse et empêchent la formation de thrombi, en particulier à la suite de néphropathies aigües ou chroniques, de glomérulonéphrites ou de coagulation intra-vasculaire.

Les dérivés de l'invention ont un effet cytotrophique sur les cellules qui participent aux défenses immunitaires de l'organisme, en particulier les macrophages, les monocytes et les lymphocytes. Ces dérivés favorisent la différenciation des lymphocytes, macrophages et monocytes ; ils augmentent la production des facteurs de l'immunité, favorisent l'acquisition de caractères différenciés et permettent de renforcer les défenses naturelles ou induites de l'immunité.

Les dérivés de l'invention ont un effet cytotrophique sur les mastocytes et les leucocytes basophiles qui jouent un rôle primordial dans les phénomènes inflammatoires. Ces dérivés favorisent l'acquisition de caractères différenciés de ces cellules, modifient leur réponse à la stimulation par un antigène et par les IgE et diminuent la production consécutive d'histamine. Ils modifient également la réponse des cellules musculaires lisses aux médiateurs de l'inflammation, comme l'histamine, les leucotriènes et les prostaglandines.

Les dérivés de l'invention ont un effet cytotrophique et différenciateur sur des cellules qui participent à la défense de l'organisme contre la néoplasie. Ils augmentent l'activité phagocytaire des macrophages, des lymphocytes et des monocytes et augmentent la sécrétion de facteurs tumoricides, comme le TNF (tumor necrosis factor). Ils ont aussi un effet différenciateur sur des cellules cancéreuses ou précancéreuses, comme les promyélocytes.

Les dérivés de l'invention ont également un effet bactériostatique. Ils empêchent la prolifération bactérienne, en particulier celle des bactéries $gram^+$ et des mycoplasmes. Lorsque l'on ajoute les dérivés de l'invention à une culture contenant des bactéries $gram^+$, comme par exemple Streptococcus mutans, Clostridium butyricum ou Streptococcus sanguis ou des mycoplasmes comme Mycoplasma pneumoniae ou Mycoplasma gallisepticum, on observe un ralentissement de leur prolifération que l'on peut mesurer par l'évolution de la turbidité du milieu de culture au cours du temps, ou par une variation de pH du milieu. On note que dans la culture contenant des alcools gras à longue chaîne de l'invention, l'augmentation de la densité optique est nettement ralentie par rapport au milieu témoin, de même que l'acidification du milieu.

Le rôle important de ces composés est conforté par le fait que l'inhibition de leur synthèse dans Clostridium butyricum entraine un ralentissement significatif de la croissance (GILBERTSON et coll., J. Lipid. Res., 19 (1978) 757-762) . Par ailleurs, la présence d'alcools gras à longue chaîne de l'invention dans le milieu est susceptible de permettre l'action bactéricide de certains antibiotiques. Ainsi, la combinaison du tridecanol et de la gentamycine entraîne une inhibition de la croissance de Streptococcus mutans (GILBERTSON et coll., Antimicrob. Agents Chemother., 26 (1984) 306-309).

Par ailleurs, les dérivés de l'invention possèdent une action anti-virale qui s'exerce principalement sur les virus à enveloppe comme le virus de l'herpès, VSV (Virus de la Stomatite Vésiculeuse), le virus rabique, les virus de type HIV (virus responsable du SIDA). Le modèle expérimental permettant de démontrer l'activité anti-virale des alcools gras selon l'invention varie selon le virus considéré. Il s'agira de sélectionner les types cellulaires sensibles au virus étudié ; on choisira donc des fibroblastes, des cellules rénales, des érythrocytes ou des cellules nerveuses.

Dans tous les cas, on observe une inhibition de l'effet cytopathogène du virus lorsqu'on ajoute des alcools gras de l'invention au milieu de culture. On mesure cet effet par des critères morphologiques ou biochimiques de dégénérescence cellulaire. On note qu'avec une même dose infectieuse, les cellules en culture sont sensiblement plus résistantes au virus lorsque le milieu contient des alcools gras à longue chaîne de l'invention. L'action anti-virale peut s'exercer soit directement au niveau de la réplication du virus à l'intérieur de la cellule, soit par un mécanisme d'inactivation qui diminue les propriétés infectieuses du virus (SNIPES et coll., Anitimicrob. Agents Chemother., 11 (1977) 98-104).

L'invention concerne également les compositions pharmaceutiques comprenant au moins un des dérivés sus-mentionnés en association avec un véhicule physiologiquement acceptable, et l'utilisation de ces compositions, notamment, dans les domaines thérapeutiques suivants :

(a) le traitement ou la prévention de la dégénérescence neuronale, en particulier au cours des maladies neurodégénératives et la perte neuronale liée à des composés chimiques ou à des acides aminés excitateurs, comme l'acide glutamique,
(b) le traitement ou la prévention des lésions du tissu nerveux chez les mammifères, qu'il s'agisse de lésions traumatiques, de lésions chimiques, de lésions dues à une maladie ou à un déficit congénital,
(c) le traitement de la maladie d'Alzheimer ou de Parkinson, la chorée de Huntington et autres maladies neurodégénératives, l'épilepsie, la dégénérescence wallérienne, le vieillissement cérébral et des maladies génétiques comme le syndrome de Down,
(d) le traitement et la prévention des lésions cérébrales liées à la neurochirurgie (l'administration de ces compositions se faisant concomitamment à la chirurgie),
(e) la cicatrisation de lésions cutanées, ainsi que le traitement et la prévention des phénomènes liés au vieillissement de la peau,
(f) la réparation de lésions vasculaires (d'origine traumatique ou chimique), ainsi que le traitement et la prévention des phénomènes de thrombose et d'athérosclérose,
(g) le traitement et la prévention de la dégénérescence du tissu hépatique, rénal ou cardiaque, en particulier lorsqu'elle est due à des composés chimiques ou médicamenteux toxiques pour ces organes,
(h) le traitement et la prévention des ulcères gastriques et duodénaux,
(i) le traitement des déficits acquis ou héréditaires de l'immunité, et la stimulation des défenses immunitaires, notamment au niveau du système nerveux central, notamment par activation des macrophages et des lymphocytes,
(j) le traitement des processus inflammatoires et allergiques aigus, comme l'asthme, la rhinite allergique, le choc anaphylactique ou l'urticaire,
(k) le traitement et la prévention de la néoplasie et de tumeurs, telles que les tumeurs hépatiques ou celles des os, et la prévention de la propagation de métastases provenant de tumeurs cancéreuses,
(l) le traitement et la prévention de toutes les pathologies liées aux bactéries gram$^+$ et aux mycoplasmes, en particulier les infections pulmonaires, uro-génitales et buccales,
(m) favoriser la greffe de tissus nerveux d'origine foetale ou adulte, ou la greffe de placenta, ou de tissus pancréatiques, ou de cellules à fonction immunitaire,
(n) le traitement et la prévention des pathologies liées aux virus à enveloppe, comme l'Herpès, la rage, le SIDA.

L'invention a également pour objet l'utilisation d'un dérivé de formule :

$$CH_3 - (CH_2)_n - CH_2OH$$

dans laquelle n varie de 5 à 38, ou un alcool aliphatique secondaire de 7 à 40 atomes de carbone, le cas échéant, substitué par un ou plusieurs groupes alcoyles, et comprenant le cas échéant une double liaison, pour l'obtention d'un médicament pour :

(a) la cicatrisation de lésions cutanées, ainsi que le traitement et la prévention des phénomènes liés au vieillissement de la peau,

(b) la réparation de lésions vasculaires (d'origine traumatique ou chimique), ainsi que le traitement et la prévention des phénomènes de thrombose et d'athérosclérose,

(c) le traitement et la prévention de la dégénérescence du tissu hépatique, rénal ou cardiaque, en particulier lorsqu'elle est due à des composés chimiques ou médicamenteux toxiques pour ces organes,

(d) le traitement et la prévention des ulcères gastriques et duodénaux,

(e) le traitement des déficits acquis ou héréditaires de l'immunité, et la stimulation des défenses immunitaires, notamment au niveau du système nerveux central, notamment par activation des macrophages et des lymphocytes,

(f) le traitement des processus inflammatoires et allergiques aigus, comme l'asthme, la rhinite allergique, le choc anaphylactique ou l'urticaire,

(g) le traitement et la prévention de la néoplasie et de tumeurs, telles que les tumeurs hépatiques ou celles des os, et la prévention de la propagation de métastases provenant de tumeurs cancéreuses,

(h) le traitement et la prévention de toutes les pathologies liées aux bactéries gram$^{+}$ et aux mycoplasmes, en particulier les infections pulmonaires, uro-génitales et buccales,

(i) favoriser la greffe de tissus nerveux d'origine foetale ou adulte, ou la greffe de placenta, ou de tissus pancréatiques, ou de cellules à fonction immunitaire.

Avantageusement, le traitement des maladies et troubles sus-mentionnés avec les dérivés de l'invention, en tant que composés neurotrophiques et neuroprotecteurs, est réalisé en combinaison avec l'utilisation de greffes de tissu nerveux au niveau de la lésion, en particulier de greffes de tissu nerveux d'origine foetale ou adulte, ou de greffes de placenta, ou de tissu pancréatique (traitement du diabète), ou encore de cellules à fonction immunitaire (traitement de cancers, notamment du cancer du rein).

Cette combinaison permet de favoriser la récupération fonctionnelle ou de protéger le tissu nerveux par divers mécanismes, comme : un effet trophique sur le tissu nerveux ; la libération de neurotransmetteurs ou d'hormones ; ou la réinnervation de certains éléments du tissu nerveux. On peut également associer à cette combinaison de greffes nerveuses et de dérivés de l'invention, des greffes de cellules gliales ou de tissu capable de produire des facteurs neurotrophiques (tels que le facteur de croissance (NGF) neuronale, ou le facteur de croissance de fibroblaste (FGF), le facteur de croissance des dérivés des plaquettes (PDGF), le facteur de croissance insulinique (IGF ou somatomédines).

Les compositions pharmaceutiques sus-mentionnées comprennent de préférence des doses efficaces de dérivés de l'invention qui sont de l'ordre de 0,01 mg/kg à 100mg/kg, notamment de 0,5 mg/kg à 10 mg/kg.

Des compositions pharmaceutiques de l'invention comprennent avantageusement une dose unique comprise entre 0,5 mg et 500 mg de dérivé de l'invention, en association avec un véhicule ou excipient pharmaceutiquement acceptable, notamment pour les voies parentérale ou orale.

La concentration optimale de ces molécules varie selon l'importance de la lésion ou la présence d'effets secondaires.

Les compositions pharmaceutiques de l'invention sont administrées par voie parentérale, notamment par voie intraveineuse, intramusculaire, intracérébrale ou intrapéritonéale ; elles sont également administrées par voie sublinguale, vaginale ou par des implants ou des pompes.

De préférence, les compositions pharmaceutiques de l'invention sont administrées par voie orale.

Les préparations parentérales sont préparées par stérilisation à travers un filtre avec des pores de 0,22 µm ; on prépare des flacons de 1 ml bouchés stérilement contenant 100 mg de ces molécules. On peut également utiliser un lyophilisat qui sera reconstitué avant injection intraveineuse.

Les compositions de l'invention adaptées pour la voie orale, se présentent avantageusement sous forme de pilules, gélules, suspensions, poudres, émulsions ou capsules.

Avantageusement, les compositions pharmaceutiques sus-mentionnées, utilisées dans le domaine de la neuroprotection, comprenent au moins un dérivé de l'invention en association avec d'autres facteurs neurotrophiques ou neuroprotecteurs, comme le NGF le FGF, les somatomédines, les benzodiazépines, les antagonistes des récepteurs kappa, les bloqueurs du canal calcium et des antagonistes des récepteurs des acides aminés excitateurs.

Des compositions pharmaceutiques particulièrement avantageuses dans le cadre de greffes cellulaires, notamment de greffes de cellules nerveuses, comprennent un, ou plusieurs dérivés de l'invention en association avec des cellules ayant les propriétés de celles originellement contenues dans la, ou les, partie(s) lésée(s) où la greffe doit être effectuée. A titre d'exemple, de telles compositions pharmaceutiques, selon l'invention, se présentent sous forme de préparations injectables comprenant une quantité déterminée d'un ou plusieurs des dérivés de l'invention en association avec des cellules provenant du septum ou de l'hippocampe (ces cellules étant prélevées sur un autre organisme que celui recevant la greffe).

Dans le cadre de l'utilisation bactériostatique, des compositions pharmaceutiques particulièrement avantageuses, comprennent un, ou plusieurs dérivés de l'invention en association avec un, ou plusieurs antibiotiques.

L'ensemble des compositions pharmaceutiques de l'invention est destinée au traitement et/ou à la prévention des pathologies décrites ci-dessus chez les mammifères.

L'invention a également pour objet des milieux de culture comprenant au moins un des composés de l'invention en association avec des produits appropriés pour la culture de cellules. Ces milieux de culture sont plus particulièrement destinés à la culture de cellules telles que les cellules humaines ou animales provenant de tissu dissocié embryonnaire d'animal nouveau-né, adulte ou âgé. Les dérivés de l'invention favorisent la survie et la différenciation de ces tissus in vitro. A titre d'exemples de cellules susceptibles d'être mises en culture dans les milieux de l'invention, on peut citer : les cellules nerveuses, les macrophages, les hépatocytes, les cellules rénales, les fibroblastes, les cellules endothéliales, les lymphocytes et les cellules de la moelle osseuse. Ces cellules peuvent être administrées à un patient dans un but thérapeutique, éventuellement après avoir subi un traitement in vitro avec un ou plusieurs dérivés de l'invention dans les milieux de culture sus-mentionnés.

A ce titre, l'invention vise également des compositions pharmaceutiques comprenant des cellules telles qu'indiquées ci-dessus, en association avec un véhicule pharmaceutiquement acceptable, issues d'une culture de cellules selon l'invention, ces cellules étant de préférence exemptes des autres constituants de ces milieux de culture.

A titre illustratif, des exemples de milieux de culture de l'invention sont plus particulièrement décrits dans la description détaillée qui suit.

D'autres caractéristiques de l'invention apparaîtront encore au cours de la description qui suit d'exemples de synthèse et des propriétés biologiques des dérivés de l'invention. Il va de soi que ces exemples ne revêtent aucun caractère limitatif.

## I EXEMPLES DE SYNTHESE DE DERIVES DE L'INVENTION

1) Dérivés saturés et insaturés de la vitamine A **Exemple A** :

a) Préparation du sel de bromure de phosphonium

Pour éviter des réactions secondaires, les bromures d'alcool primaire de longueur de chaîne convenable Br $(CH_2)_n$-OH sont traités par le 4 methoxy -3-4 dihydropyrane afin de protéger l'hydroxyle primaire.

Le sel de bromure de phosphonium est préparé par l'addition de 0.4 moles de triphenylphosphine dans une solution de 0.4 moles de bromure de methoxytetrahydropyrane dans 100 ml de toluène.

La solution est ensuite chauffée à 60°C pendant 1 nuit. Le sel de bromure de phosphonium précipite au cours du refroidissement, il est filtré et lavé avec 100 ml de toluène.

b) Réaction de Wittig

A une suspension de 2 mmoles de sel de bromure de phosphonium obtenu de la manière indiquée ci-dessus, dans 9 ml d'hexane anhydre , on ajoute goutte à goutte 0,8 ml (1,26 mmoles) de n-butyllithium (hexane) on laisse réagir à la température ambiante pendant 1 nuit.

On ajoute ensuite goutte à goutte 0,6 mmoles de rétinal dissous dans 2 ml d'hexane. Après 1 heure de réaction, on refroidit avec un bain de glace et on filtre pour enlever le triphénylphosphate formé. Après évaporation on obtient l'oléfine formée avec un rendement de 80 %.

L'oléfine (0,5 mmoles ) dissous dans 30 ml de méthanol est hydrogénée en présence de 50 mg de Palladium sur charbon (Pd/c) à 5 %. On obtient quantitativement le produit désiré lié au méthoxytetrahydropyrane. Le groupe protecteur est éliminé par un simple traitement avec une solution de HCl à 0,1 N.

Le nombre de liaisons hydrogénées est déterminé par la durée de la réaction. On peut également protéger certaines double-liaisons que l'on ne désire pas saturer, notamment par la méthode de protection au dibromure.

**Exemple B : élongation de chaîne d'un, de deux ou de trois atomes de carbone à partir du rétinol perhydrogéné :**

B-I. Rétinol perhydrogéné + un atome de carbone 1. Réaction :

METHODE DE SYNTHESE (1)

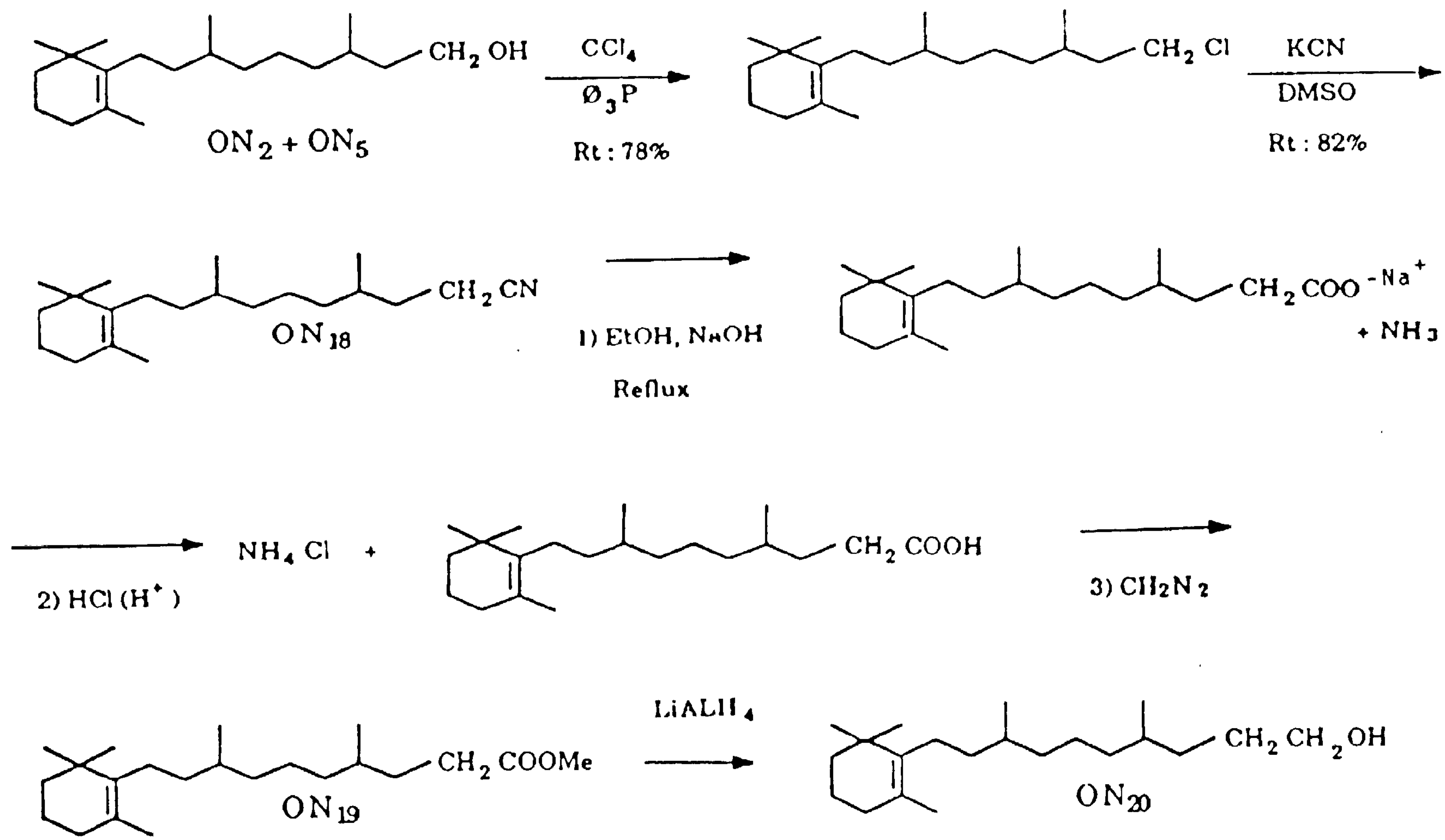


$ON_2 + ON_5$

$ON_{18}$

$ON_{19}$

$ON_{20}$

1), 2) et 3) sont effectués directement sans purification

2. Mode opératoire :

a. Chloration

Réaction anhydre, $CCl_4$ distillé sur $P_2O_5$. Le rétinol perhydrogéné (200 mg) dissous dans 20 ml de $CCl_4$ est placé dans un ballon. On ajoute ensuite $\phi_3P$ en léger excès. La réaction se fait sous agitation, sous argon et sous reflux à 85°C. Durée de la réaction : 24 heures. On place ensuite le ballon dans de l'eau + glace pour que $\phi_3P=O$ précipite. $\phi_3P=O$ est ensuite éliminée par filtration.

b. Synthèse du nitrile

Réaction anhydre. DMSO distillé sur $CaH_2$ sous vide. On place dans un ballon du KCN (2 équivalents) + DMSO (20 ml). Le mélange est chauffé sous argon à 100°C jusqu'à la dissolution complète de KCN. On y ajoute ensuite une solution du chlorure ($4.10^{-3}$ moles) précédemment obtenue dans 5 ml de DMSO. On monte à 120°C. Durée de la réaction : 6 heures. On ajoute ensuite NaCl saturé + éther et on extrait à l'éther; on lave à l'eau.

c. Synthèse de l'ester

Dans un ballon, on place le nitrile ($3.10^{-3}$ moles) dissous dans 15 ml d'éthanol + 5 ml d'une solution de NaOH 12N. On laisse sous agitation et sous argon et sous reflux pendant toute la nuit. On évapore EtOH et on dilue le résidu dans $H_2O$ et on extrait les impuretés à l'éther. On ajoute HCl concentré goutte à goutte dans la phase organique jusqu'à PH = 2. On extrait à l'éther et on lave la phase organique avec NaCl + $H_2O$. L'éther est ensuite évaporé. On dissout le résidu dans l'éther et on ajoute goutte à goutte une solution éthérée de $CH_2N_2$ sous agitation dans un bain de glace + eau. On ajoute de l'acide acétique concentré pour détruire l'excès de $CH_2N_2$ dès que la réaction est terminée (contrôlé sur CCM). On lave avec NaCl. Durée totale : 10 heures. Rendement total : 90%.

d. Réduction de l'ester en alcool

Dans un ballon, on place l'ester ($3{,}5.10^{-3}$ moles) dissous dans l'éther (50 ml) + 230 mg de LiA1H4. La réaction est très exothermique avec dégagement d'H2. Elle est instantanée. On ajoute 5 ml d'une solution de $H_2SO_4$ 10N pour détruire l'excès de $LiA1H_4$. Extraire avec $CH_2Cl_2$ et laver la phase organique à l'eau.

B-II. Rétinol perhydrogéné avec deux atomes de carbone :

1. Réaction :

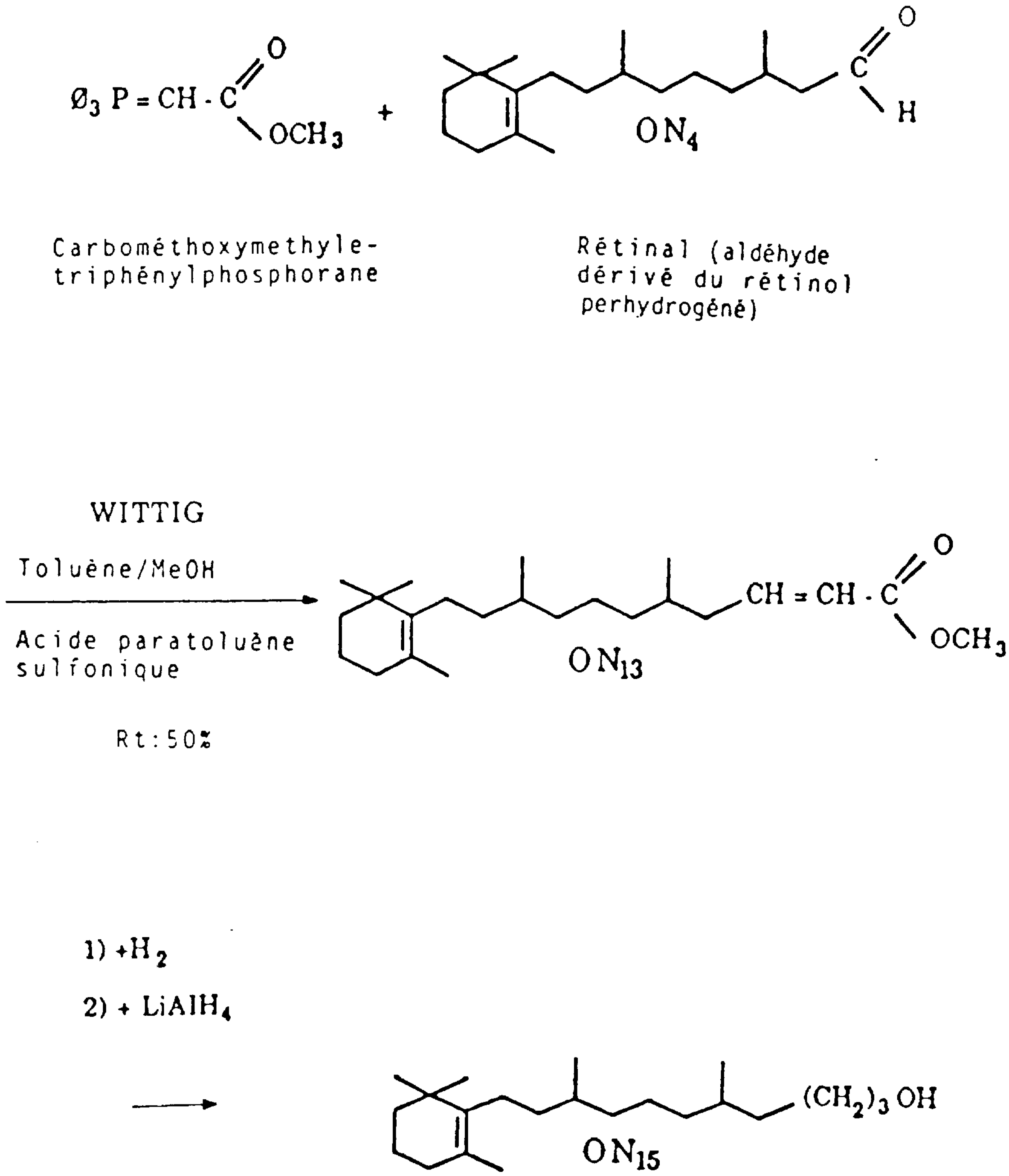


ON4

Carbométhoxymethyle-triphénylphosphorane

Rétinal (aldéhyde dérivé du rétinol perhydrogéné)

ON13

ON15

2. Mode opératoire :

Préparation du rétinal

a. Réaction anhydre. $CH_2Cl_2$ distillé sur $CaH_2$

Dans un ballon, on place $5.10^{-3}$ moles de pyridinium chloromate + 10 ml de $CH_2Cl_2$. On obtient une suspension à laquelle on ajoute $1,6.10^{-3}$ moles de rétinol dissous dans 5 ml de $CH_2Cl_2$. La suspension devient alors noire. Le mélange est agité sous argon. Durée de la réaction : 4 heures. Rendement : 70%.

b. Dans un ballon, on place quelques grains d'acide paratoluène sulfonique sec + $1,7.10^{-3}$ moles de (Carbomethoxy-methylène)triphénylphosphorane + toluène distillé (15 ml). On y ajoute le rétinal dissous dans 5 ml de toluène et

du méthanol (700 µl) pour dissoudre l'ylure. Le mélange est chauffé sous argon à 95°C. Durée de la réaction : 3 jours. On arrête la réaction par addition d'une solution saturée en $NH_4Cl$. L'ester obtenu est réduit en alcool par action de $LiA1H_4$ comme il est décrit ci-dessus. On extrait la phase organique à l'éther. On lave à l'eau et on évapore l'éther ethylique.

B-III. Rétinol perhydrogéné avec trois atomes de carbone :

1. Réaction :

METHODE DE SYNTHESE (2)

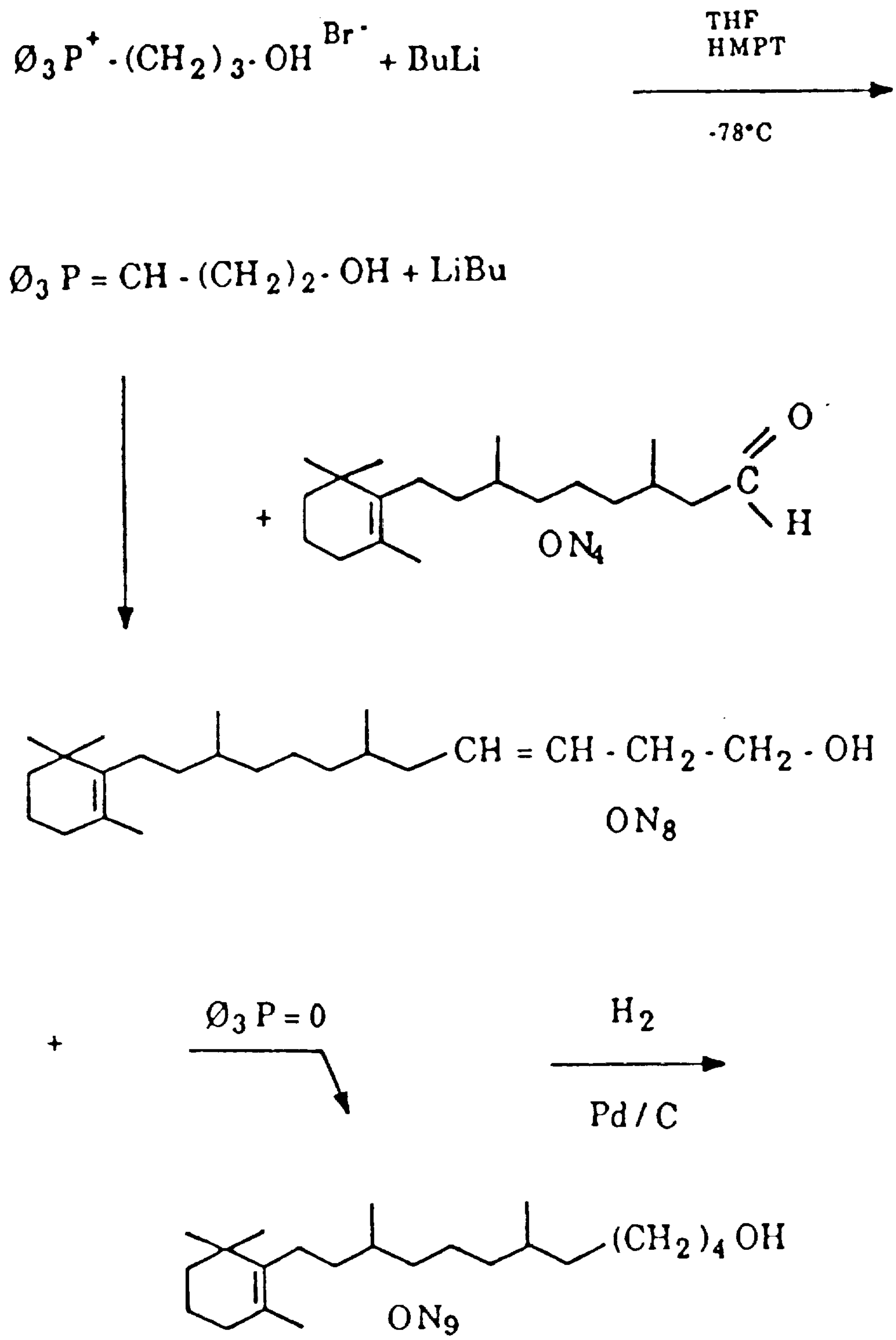


ON4

ON8

ON9

2. Mode opératoire :

a. Réaction anhydre. THF et HMPT secs

On place le sel de phosphonium du 3 bromopropanol ($4.10^{-4}$ moles) sec dans un ballon. On additionne THF (5 ml) et HMPT (0.5 ml). On met l'agitation et on descend à -78°c. On ajoute alors trois équivalents de BuLi. On place ensuite le ballon à -30°C et on laisse agir pendant une heure environ. Quand le sel est entièrement dissous, on redescend à -78°C, on ajoute le rétinal perhydrogéné ($8.10^{-4}$ moles) et on laisse revenir à température ambiante toute une nuit. Rendement : 38%. On arrête la réaction par addition d'une solution saturée en $NH_4Cl$. On extrait l'éther puis on lave à l'eau.

b. Dans un ballon, on met le produit à hydrogéner ($2.10^{-4}$ moles) dissous dans 10 ml de MeOH pur. On ajoute du palladium sur carbone (10 mg). Puis on fait passer de l'hydrogène. On filtre le Pd/C sur célite et on évapore le méthanol.

Des dérivés de la vitamine A ainsi synthétisés présentent les caractéristiques analytiques suivantes:

| Produit | PM | Rf | solvants d'élution |
|---|---|---|---|
| 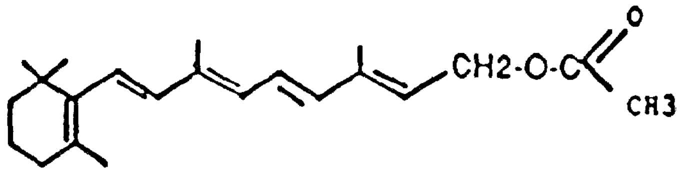 n°1 | 330 | 0,42 | (TEA, Et2O, Hexane) (1, 10, 90) |
| 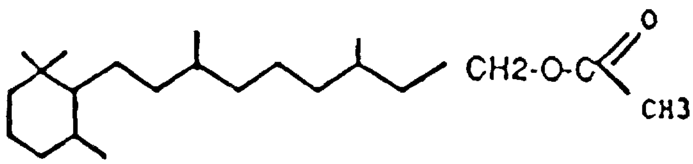 N°2 | 340 | 0,547 | ( " " " )( " ) |
| 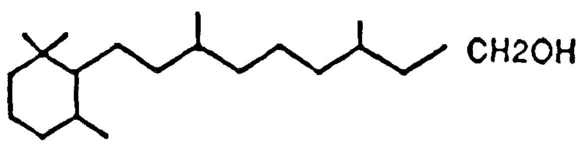 n°3 | 298 | 0,573 | (TEA, Et2O, Hexane) (1, 60, 40) |
| 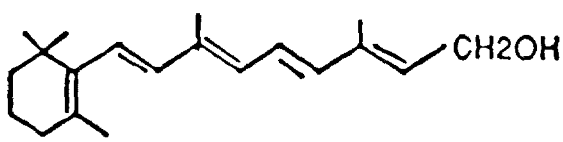 n°4 | 288 | 0,28 | (TEA, Et2O, Hexane) (1, 60, 40) |
| 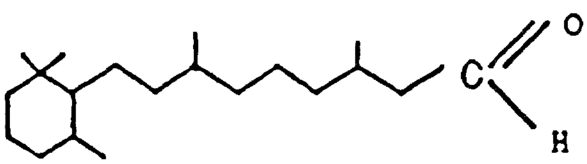 n°5 | 296 | 0,8 | (TEA, Et2O, Hexane) (1, 50, 50) |

TEA : triéthylamine

| Produit | PM | Rf | solvants d'élution |
|---|---|---|---|

Dérivés de la vitamine A

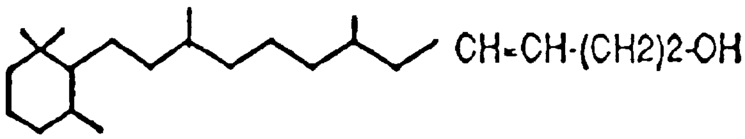

| n°5 | 336 | 0,54 | (Hexane, ether)(50/50) |
|---|---|---|---|

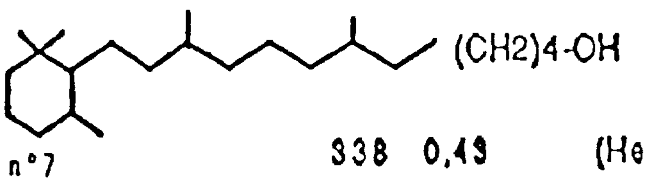

| n°7 | 338 | 0,43 | (Hexane, ether)(60/50) |
|---|---|---|---|

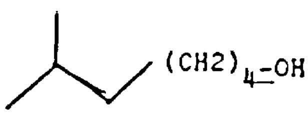

| ON7 : $C_8H_{16}O$ | 128 | 0,375 | (Hexane, ether)(60/40) |
|---|---|---|---|

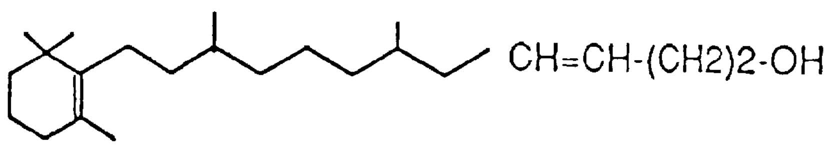

| ON8 : $C_{23}H_{42}O$ | 335 | 0,403 | (Hexane, ether)(60/40) |
|---|---|---|---|

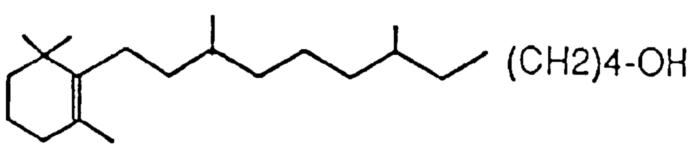

| ON9 : $C_{23}H_{44}O$ | 337 | 0,58 | (Hexane, ether)(40/60) |
|---|---|---|---|

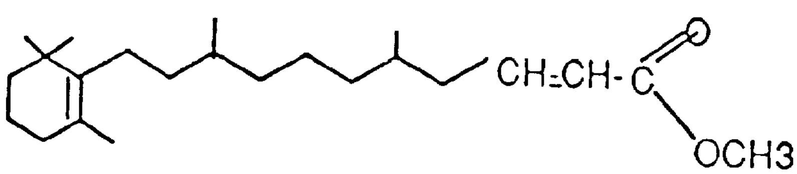

| Produit | PM | Rf | solvants d'élution |
|---|---|---|---|
| ON13 : $C_{23}H_{40}O_2$ | 349 | 0,326 | (Hexane, ether)(90/10) |

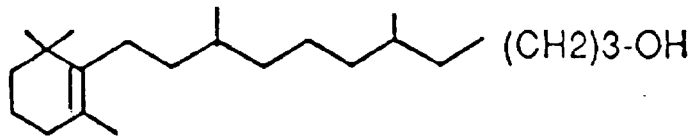

| Produit | PM | Rf | solvants d'élution |
|---|---|---|---|
| ON15 : $C_{22}H_{44}O_2$ | 323 | 0,455 | (Hexane, ether)(60/40) |

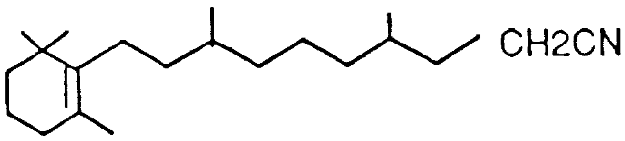

| Produit | PM | Rf | solvants d'élution |
|---|---|---|---|
| ON18 : $C_{21}H_{37}N$ | 304 | 0,574 | (Hexane, ether)(80/20) |

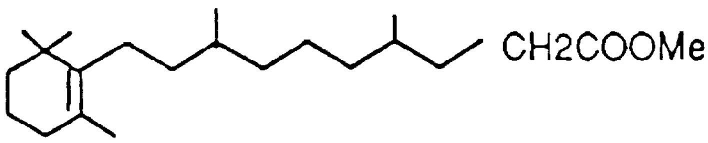

| Produit | PM | Rf | solvants d'élution |
|---|---|---|---|
| ON19 : $C_{22}H_{40}O_2$ | 337 | 0,750 | (Hexane, ether)(80/20) |

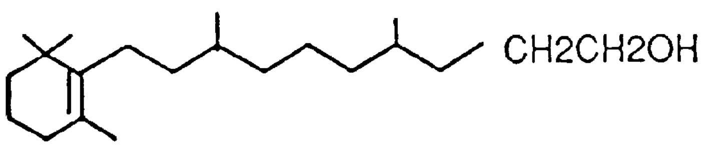

| Produit | PM | Rf | solvants d'élution |
|---|---|---|---|
| ON20 : $C_{21}H_{40}O$ | 309 | 0,237 | (Hexane, ether)(80/20) |

**ON21**

- Formule brute : $C_{21}H_{38}O$
- Formule développée :

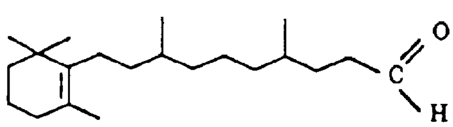

- Poids moléculaire : 306,5 g.mol$^{-1}$
- Rf : 0,707 avec 80% hexane / 20% ether

**$ON_{22}$**

- Formule brute : $C_{24}H_{44}O$
- Formule dévelopée :

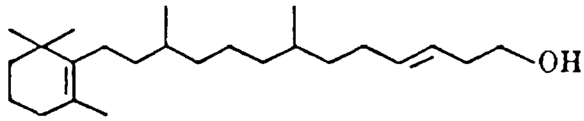

- Poids moléculaire : 348,6 $g.mol^{-1}$
- Rf: 0,450 avec 60% hexane / 40% ether

**$ON_{23}$**

- Formule brute : $C_{24}H_{46}O$
- Formule développée :

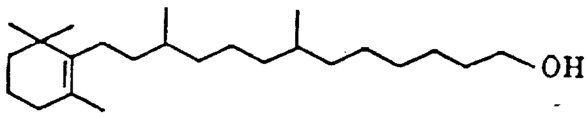

- Poids moléculaire : 350,6 $g.mol^{-1}$
- Rf : 0,29 avec 80% hexane / 20% ether

Les deux dérivés hydrogénés de la molécule n°4 définie ci-dessus, répondant aux formules suivantes :

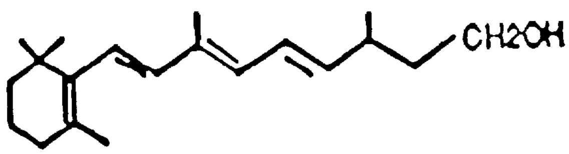

PM 290

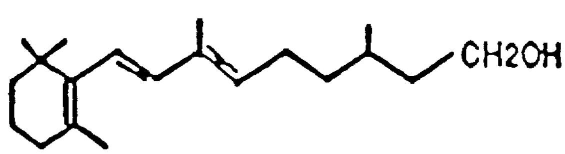

PM 292

ainsi que les dérivés n° 6 et n° 7, ON7 à ON9, ON13, ON15, ON18 à ON20, sont des dérivés particulièrement actifs dans le cadre de la présente invention, notamment dans le test décrit dans l'exemple 1 (effet trophique sur des neurones in vitro) du Chapitre II qui suit.

2) Dérivés du squalène

**Réaction : Ozonisation du squalène**

Une solution de 4 mmoles de squalène dans 100 ml de CH2Cl2 et 1 ml de pyridine est traitée par $O_3$ à -70°C pendant 2 heures. Après addition de 1 ml de $(CH_3)_2S$, le mélange de réaction est laissé réagir pendant 1 heure à -70°C puis 1 heure à 0°C puis est extrait avec $CHCl_3$. Après l'évaporation du solvant, le résidu est chromatographié sur $SiO_2$. On obtient en particulier deux produits d'activité biologique désiré: il s'agit d'un alcool à 27 atomes de carbone et un diol à 24 atomes de carbone.

3) Dérivé hydrogéné du géraniol (C20)

Le dérivé de formule $C_{15}H_{31}$-$CH_2OH$ a été préparé par hydrogénation du géraniol.
Ses caractéristiques analytiques sont les suivantes :

PM = 242,
Rf = 0,454 dans le solvant d'élution, $Et_2O$ -Hexane (50, 50).

4) Composés dimériques.

Un caroténoïde contenant deux groupes hydroxyles sur les deux extrémités de la molécule, le decaprenyl astaxanthine, est hydrogéné sur Pd/C avec l'éthanol comme solvant.

5) Dérivés hydrosolubles des alcools à longue chaîne : esters de l'acide monophosphorique.

**a) monoester de l'acide monophosphorique**

Cette réaction est valable pour les alcools linéaires, comme pour les dérivés et analogues des terpènes et les esters du cholestérol de l'invention comprenant une fonction hydroxyle, de préférence située à l'extrémité d'une chaîne hydrocarbonée. Pour la compréhension de l'exposé qui suit, l'ensemble des dérivés cités ci-dessus, seront désignés par l'expression simplifiée $R_6$-OH faisant apparaître l'hydroxyle impliqué dans la réaction de phosphorylation et dans laquelle $R_6$ représente le reste de ces dérivés, à savoir plus particulièrement une chaîne hydrocarbonée du type $R_1$-[A-$(X)_y$-B$]_p$- telle que définie ci-dessus,

On utilise un mode de préparation permettant d'obtenir sélectivement le monoester de l'acide monophosphorique. Ce mode opératoire fait intervenir le bis(2,2,2 trichloroethyl) phosphochloridate comme réactif et le couple Zn/Cu comme agent de déprotection pour libérer le sel de phosphate.

Préparation du réactif

Le réactif bis(2,2,2 trichloroethyl) phosphochloridate (BTEP) est préparé par addition goutte à goutte pendant 15 minutes à -50°C du trichloroethanol (44,8 g soit 29 ml ou 0,3 mole). Puis le mélange est laissé à réagir à 0°C pendant 2 heures sous argon. Le mélange de réaction est chauffé à 55°C pendant une nuit et distillé pour donner 16,7 g (0,046 mole soit 15,3 %) de bis(2,2,2 trichloroethyl) phosphochloridate Pel=98°C (0,15 mm Hg), $d_4^{15}$ = 1,75 g/ml.

Phosphorylation

L'alcool ($R_6$-OH) dissous dans 2 ml de THF (0,1 mmole) est refroidi à -78°C. On ajoute 750 mg de tamis moléculaire 4A puis 43 µl (soit 0,21 mmole) de BTEP. La température est laissée monter à la température ambiante ; puis le tamis moléculaire est éliminé par filtration. Le filtrat est dilué à l'éther éthylique (10 ml). Puis on y ajoute 5 ml d'une solution à 0,5 M de NaHC03 suivie d'une solution éthérée d'iode (38 mg, 0,3 mmole dans 5 ml d'éther éthylique). L'addition se fait lentement goutte à goutte avec une forte agitation. Au bout de 20 minutes de réaction, on ajoute une solution aqueuse de $Na_2SO_3$ pour détruire l'excès de l'iode. On lave la solution avec une solution aqueuse saturée en NaCl et on évapore sous vide. Une chromatographie sur $SiO_2$ avec du chloroforme-méthanol (50:1) comme éluant donne le phosphate de triester avec un rendement de 92 %.

Elimination du groupement trichlorethyle.

A une solution du phosphate de triester précédemment obtenu (0,05 mmole) dissous dans 1 ml de DMF, on ajoute

65 mg de Zn/Cu et 50 µl (0,5 mmole) d'acétylacétone. La solution est chauffée à 55°C pendant 1 heure. Le mélange est agité en présence de la résine Chelex pendant 1 heure ; puis on élimine la résine par filtration. Le filtrat est évaporé à sec. Le résidu est traité par l'acide formique (300 µl) et laissé réagir pendant 24 heures, puis évaporé à sec et enfin chromatographié sur DEAE Sephadex (colonne 0,9 x 12 cm) avec un tampon pH 7,5 comme éluant. On obtient alors le phosphate avec un rendement de 75 %,

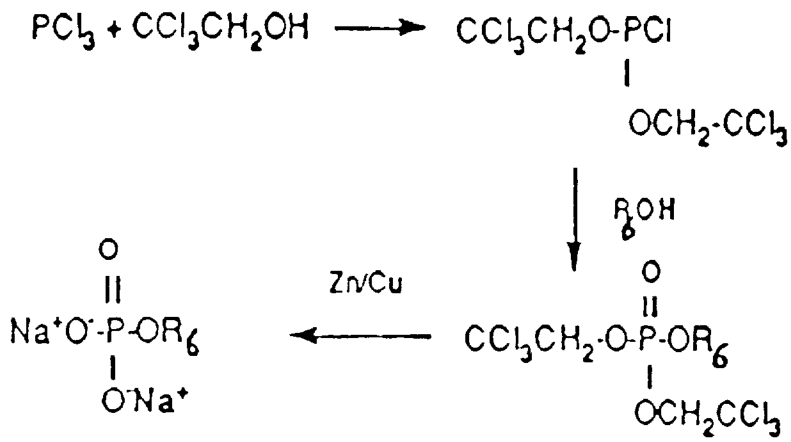


**b) diester de l'acide monophosphorique**

Cette réaction procède par phosphorylation avec l'o-phenylènephosphochloridate suivie d'une hydrolyse oxydante et permet d'obtenir les diesters de l'acide monophosphorique. Cette réaction est valable pour les alcools linéaires, comme pour les dérivés et analogues des terpènes et les esters du cholestérol de l'invention, c'est-à-dire les dérivés de formule $R_6$-OH mentionnés dans le paragraphe précédent. Dans ce cas, le groupe $R_6$ représente de préférence une chaîne hydrocarbonée de 8 à 28 atomes de carbone, le cas échéant substituée par un ou plusieurs atomes d'hydrogène, notamment de fluor, ou par un ou plusieurs, groupe(s) alcoyle(s) de 1 à 3 atomes de carbone.

**1ère étape : phosphorylation des alcools**

Une solution d'alcool contenant 1 mmole de dérivé de l'invention (383 mg dans le cas de l'hexacosanol), représenté par l'expression $R_6$-OH ($R_6$ représentant le reste du dérivé de l'invention, à savoir une chaîne alkyle de 26 atomes de carbone dans le cas de l'hexacosanol), et du 2,6 dimethylpyridine (110 mg, 1 mmole) est traitée par une solution de 100 mg (0,5 mmole) d'o-phenylène phosphochloridate dans du THF pendant 24 heures à 40°C. Après addition d'une solution saturée de $NaHCO_3$ (10 ml) l'extraction se fait avec $CH_2Cl_2$ et la chromatographie sur $SiO_2$ donne 330 mg de phosphate (85 %).

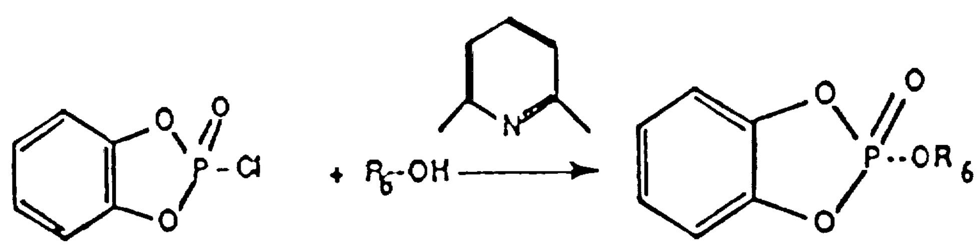

**2ème étape : hydrolyse suivie de l'oxydation**

Le produit précédemment obtenu (330 mg) dissous dans 6 ml de THF est traité par une solution de 110 mg (1 mmole) de dimetylpyridine dans 200 ml d'eau pendant 5 minutes, puis extrait avec du chloroforme ($CHCl_3$). L'extrait à $CHCl_3$ est dissous dans du THF (10 ml) puis traité avec 1g de téraacétate de plomb ($Pb(OAc)_4$) ; le mélange est ajusté à pH 10 avec une solution méthanolique 0,1N de potasse (KOH). Après une évaporation du solvant et lavage avec du

méthanol (MeOH), le sel de potassium est obtenu sous forme de liquide brun qu'on purifie par chromatographie sur $SiO_2$ avec comme éluant $CHCl_3/MeOH/H_2O$ 60:25:5.

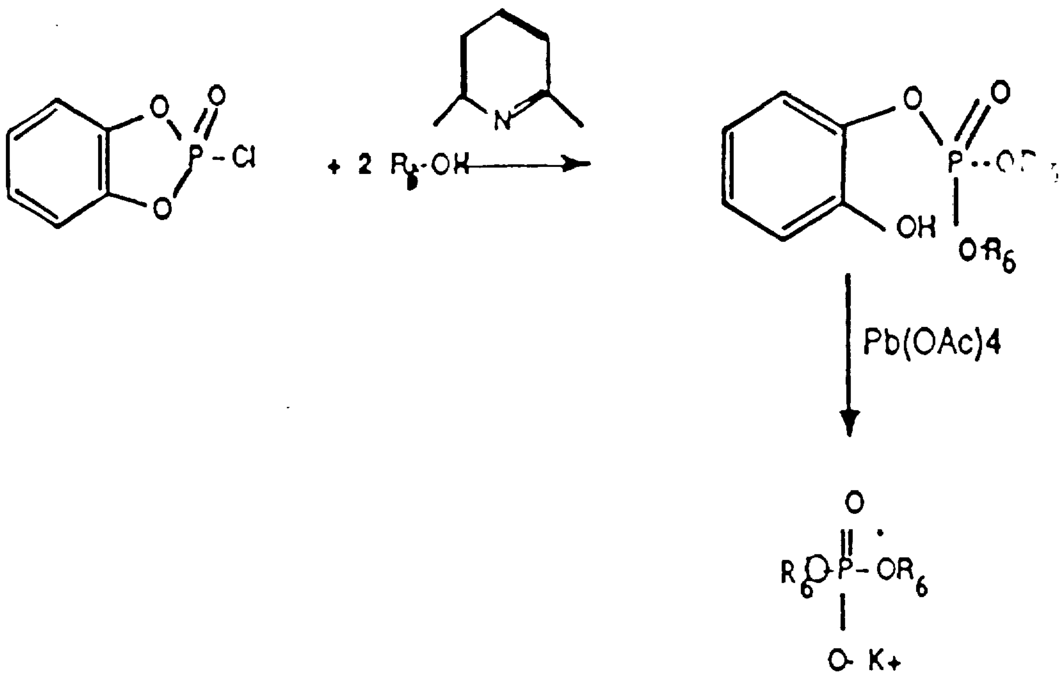


L'ester de l'acide monophosphorique de formule

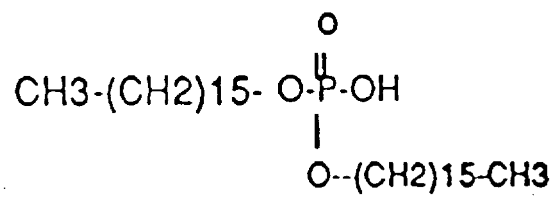

ainsi obtenu possède un PM de 547 et un Rf de 0,375 dans le solvant d'élution : $CHCl_3$, Methanol, $H_2O$ (65, 25, 4).

6) Substitution par des atomes de fluor

Il a été démontré précédemment que des composés organofluorés peuvent potentialiser l'effet des composés biologiques (Y. KOBAYASHI, I. KUMADOKI et T. TAGUCHI, Synthesis of biologically active fluorine compounds. Synth. Org. Chem., 38 (1980) 1119-1129). Par des techniques déjà décrites d'incorporation d'atomes de fluor (KOBAYASHI et coll., supra; J. T. WELCH, advances in the preparation of biologically active organofluorine compounds. Tetrahedron, 43 (1987) 3123-3197; Biomedical aspects of fluorine compounds, R. FILLER and Y. KOBAYASHI eds, KODANSHA, Tokyo, (1982)), on montre que la substitution de 1 à 5 atomes de fluor sur des alcools gras à 26 atomes de carbone ± 6 améliore la biodisponibilité des molécules.

La fluorination se fait par un mécanisme électrophilique.

La substitution peut se faire sur une liaison saturée C-H (D. BARTON et D. OLLIS, in Comprehensive Organic Chemistry, J.F. STODDART, ed. Pergamon Press (1979) pp504-507). L'attaque électrophilique se situe directement au niveau de la liaison C-H.

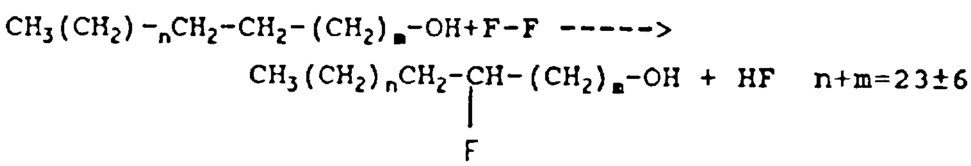

La substitution peut également se faire en saturant un ou des systèmes dessaturés par des composés contenant un ou plusieurs atomes de fluor.

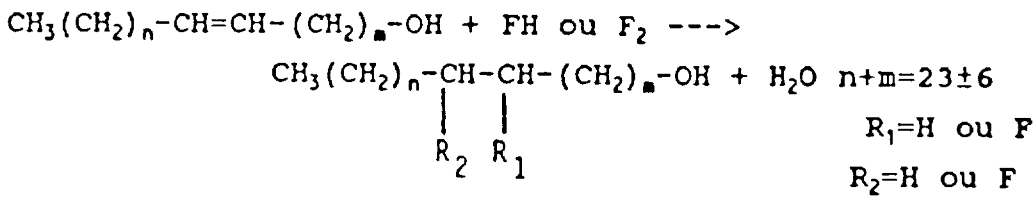


Les composés fluorés contenant de 1 à 5 atomes de fluor améliorent la biodisponibilité de la molécule et augmentent leur efficacité biologique.

Des dérivés difluorés sont particulièrement intéressants sur le plan des activité biologiques (T. MORIKAWA et al, Studies on Organic Fluorine Compounds. Synthesis of 2,2 difluoro arachidonic acid. Chem. Pharm. Bull, 37 (3) 813-815 (1989)). On les obtient de la façon suivante :

**Réaction détaillée :**

A un mélange de Zn (33,35 Masse atomique) et de THF (30 ml), on ajoute goutte à goutte une solution d'aldéhyde en $C_{24}$ (32 mmole) et du bromodifluoroacetate d'ethyle (28,04 mmole) dans du THF (33 ml). On chauffe sous reflux pendant 3 heures avec agitation. Après addition d'éther et de $NH_4Cl$ aqueux, le précipité est enlevé par filtration et le filtrat est extrait à l'éther, l'extrait est lavé avec une solution de NaCl puis chromatographié sur $SiO_2$. Le composé est ensuite traité par une solution de HCl 1N et hydrogéné en présence de $PtO_2$. On obtient l'ester éthylique de l'acide en $C_{26}$ avec un rendement de 57 %. Cet ester est ensuite réduit par $LiAlH_4$ dans l'éther éthylique avec chauffage sous reflux.

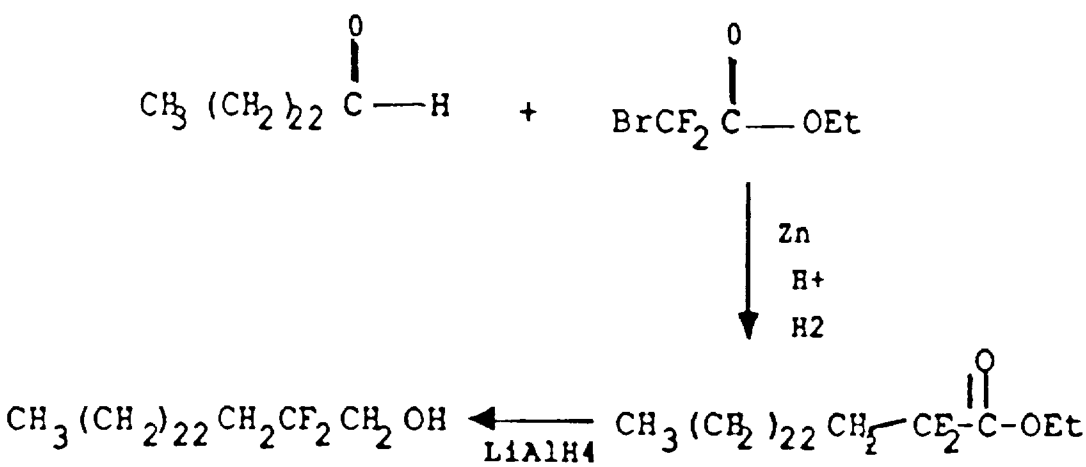


PM = 316 à 502.

**II ESSAIS BIOLOGIQUES IN VITRO et IN VIVO**

1) Régénérescence nerveuse

Les dérivés et analogues des alcools gras à longue chaîne de l'invention jouent un rôle dans le contrôle de la plasticité du tissu nerveux au cours du développement et chez l'adulte. Ils augmentent la survie neuronale au cours de phases de développement qui voient la mise en place de nouvelles connections neuronales, également chez l'adulte au cours des processus d'apprentissage et de mémoire; Comme nous le décrivons dans l'Exemple 1 qui suit, les dérivés de l'invention favorisent la différenciation neuronale ainsi que la survie des neurones. Cette propriété leur donne la capacité d'augmenter la survie de neurones lésés à la suite d'une traumatisme. Les dérivés et analogues des alcools gras à longue chaîne de l'invention favorisent la survie et la régénérescence de cellules nerveuses à la suite d'un traumatisme du cerveau ou de la moelle épinière. Ces molécules sont capables de limiter l'extension des lésions, de réduire la dégénéréscence wallérienne et favorisent la régénérescence axonale. En particulier, les molécules décrites diminuent la nécrose post-ischémique, réduisent l'oedème autour de la lésion, augmentent la perfusion sanguine du tissu nerveux atteint et limitent la prolifération de fibroblastes ou d'astroblastes au niveau de la lésion.

Ces propriétés sont démontrées à l'aide de neurones maintenus en culture primaire dans différentes conditions, ou in vivo à l'aide de divers modèles de lésion expérimentale chez l'animal (GAGE et al, Brain Res., 268 : 27-37 (1983) ; WILLIAM et al, Proc. Natl. Sci. 83 : 9231-9235 (1986)). Les doses utilisées sont de l'ordre de 0,01 mg/kg, plus particulièrement 0,1 mg/kg à 5 mg/kg. En raison de la meilleure biodisponibilité des molécules que nous décrivons et de leur plus grande efficacité au niveau de la membrane cellulaire, nous améliorons avec des doses moindres les effets précédemment observés avec le n-hexacosanol (BORG et al, Neurosci. Lett, 213 : 406-410 (1987)).

Par ailleurs, les dérivés de l'invention favorisent également la régénérescence des nerfs périphériques. A la suite d'un traumatisme du nerf périphérique, il peut y avoir perte de la continuité nerveuse, suivie de la dégénéréscence wallérienne. L'application des dérivés de la présente invention permet dans ce cas de réduire l'importance des lésions et de faciliter la restauration de la continuités nerveuse. Ce résultat est obtenu grâce aux propriétés neurotrophiques et régénérantes de ces dérivés.

**EXEMPLE 1 : SURVIE ET REGENERESCENCE DE NEURONES CEREBRAUX EN PRESENCE DE DERIVES ET ANALOGUES DES ALCOOLS GRAS A LONGUE CHAINE DE L'INVENTION.**

L'utilisation de neurones cérébraux maintenus en culture primaire permet de mettre en évidence l'activité de facteurs neurotrophiques (G. BARBIN, I. SELAK, M. MANTHORPE et S. VARON ; Neuroscience, 12 (1984) 33-43). On prépare des cultures neuronales à partir du cerveau d'embryons de rat de 13 à 18 jours. Les cellules sont ensemencées à faible densité (10 000 à 100 000 cellules par ml) ; elles poussent dans des boites de Pétri recouvertes d'un support de polylysine, en présence d'un milieu de culture qui est préparé à partir du milieu de Eagle-Dulbecco (R. DUBELCCO et G. FREEMAN, Virology, 8, (1959) ; 396) auquel on ajoute des suppléments d'insuline, de transferrine, de progestérone, de sélénium et de putrescine (BORG et al, Develop Brain Res. 18 : 37-49 (1985)). Les dérivés testés sont ajoutés au début de la culture à des doses variant de $10^{-8}$ à $10^{-5}$M.

On suit l'évolution de la culture à l'aide d'un microscope à contraste de phase. La coloration au bleu trypan permet de mesurer le nombre de neurones qui émettent des prolongements. On observe que dans les cultures contrôles le nombre de neurones vivants et de neurones avec prolongements diminue à partir d'une certaine durée de culture. Au contraire, dans les cultures traitées avec les dérivés de l'invention, le nombre de ces neurones reste constant avec le temps et la longueur des prolongements est beaucoup plus importante que dans les témoins. On note également un grand nombre de ramifications à partir de ces prolongements et la présence de nombreux contacts cellulaires. In vivo, ces observations correspondent à la mise en place de nouveaux circuits neuronaux et à la formation de jonctions intercellulaires ou synaptiques.

L'augmentation de la survie de neurones placés dans des conditions critiques et l'amélioration de leur maturation morphologique se traduit également par une maturation accrue sur le plan biochimique.

On peut mesurer la présence de certaines protéines spécifiques de ces neurones, comme les protéines des neurofilaments, l'énolase neurospécifique ou la glutaminase par des méthodes immunocytochimiques ou des méthodes enzymatiques. Après fixation on incube les cultures avec un anticorps spécifique contre un neurofilament ou contre l'énolase, à différents temps de culture. On révèle la présence de ces protéines par une méthode à la péroxidase ou avec des anticorps fluorescents. L'apparition de ces protéines est plus précoce et plus intense dans les cultures contenant les dérivés de l'invention.

On peut aussi mesurer l'activité enzymatique de l'énolase ou de la glutaminase qui sont de bons marqueurs de la maturation neuronale. (BORG et al, Neurosci. Lett., 213 :406-410 (1987)). L'activité enzymatique rapportée à la quantité de protéine permet de comparer l'effet neurotrophique des différents dérivés de l'invention. Cette mesure quantitative montre que l'effet s'observe de façon précoce et continue ; elle permet également de faire des corrélations entre la dose utilisée et l'effet observé. Dans tous les cas, les neurones traités par les dérivés et analogues des alcools gras à longue chaîne de l'invention induisent une activité accrue des marqueurs de la maturation par rapport aux contrôles.

Les dérivés de l'invention ont un effet plus important que les autres facteurs neurotrophiques déjà connus, en raison de leur meilleure biodisponibilité et d'une meilleure efficacité au niveau cellulaire. Ces résultats montrent que les dérivés et analogues des alcools gras à longue chaîne favorisent la survie de neurones cérébraux et augmentent leur degré de maturation.

Des exemples d'augmentation de la survie neuronale réalisés à l'aide des dérivés ON13, ON9 et ON8 sont représentés sur la Figure 1.

2) Neuroprotection

Une autre application intéressante des dérivés de l'invention est liée à leur propriété de protéger les neurones de lésions dues à la présence de neurotoxiques, comme le glutamate, l'acide iboténique, l'AF-64, la scopolamine ou le N-méthylD-aspartate. Le neurotransmetteur excitateur, le glutamate, et les excitotoxines en général, produisent des effets neurotoxiques en relation avec leur action dépolarisante au niveau de leurs récepteurs. Certains analogues du glutamate, comme l'acide iboténique ou le quisqualate ont des effets excitateurs plus puissants et produisent également des effets neurotoxiques. Les propriétés neuroprotectrices des dérivés et de l'invention sont démontrées à la suite d'une injection de neurotoxique, l'acide iboténique, dans le nucleus basalis chez le rat (Exemple 2). Cette injection provoque

des troubles du comportement, avec une perturbation des capacités de mémoire et d'apprentissage et une hyperactivité locomotrice. Sur le plan biochimique cette lésion entraîne une diminution de la production d'acétycholine et de l'activité de la choline acétyl transférase dans le néocrotex ipsilatéral, ainsi que des lésions morphologiques dans cette zone de cerveau. Rappelons que ce type de lésions de retrouve chez les patients souffrant de la maladie d'Alzheimer (WENK et al, Brain Res., 293 : 184-186 (1984)). Ces lésions sont sensiblement réduites chez les animaux qui ont reçu des injections intrapéritonéales à une dose de 1 mg/kg/jour de dérivés de l'invention.

Les dérivés et analogues des alcools gras à longue chaîne de l'invention peuvent donc être utilisés dans le traitement de maladies neurodégénératives, comme la chorée de Huntington, la maladie d'Alzheimer ou de Parkinson. Ils permettent également d'améliorer le traitement de l'épilepsie et d'autres troubles du comportement liés au système limbique. De plus, elles sont capables d'induire une protection des neurones vis-à-vis des neurotoxines, de certains ions et de radicaux oxydants. Pour l'ensemble de ces applications, les doses thérapeutiques sont de l'ordre de 0,1 mg/kg/jour à 10 mg/kg/jour. L'administration est répétée, par voie parentérale ou orale.

**EXEMPLE 2 : PROTECTION VIS-A-VIS DE LESIONS DUES AUX NEUROTOXIQUES**

On utilise les propriétés excitotoxiques d'un acide aminé, l'acide iboténique, pour étudier la survie des neurones cholinergiques du noyau basalis, selon une méthode précédemment décrite (WENK et al, Brain res., 293 : 184-186 (1984)). Après avoir anesthésié des rats mâles Long-Evans on les places dans un appareil stéréotaxique ; puis on injecte 25 nmole d'acide iboténique dans le noyau basalis du côté gauche.

Un groupe de rats est traité avec un dérivé d'alcool gras à longue chaîne, à raison d'une injection par jour (1 mg/kg) par voie intrapéritonéale à partir de deux jours avant l'injection d'acide iboténique.

On peut utiliser plusieurs types de tests cognitifs et de comportement au cours de l'expérience, en particulier le labyrinthe radial à 8 branches, l'apprentissage du labyrinthe de Hebb-Williams, ou le test de la piscine (ALFORD et al, Exp. Brain Res., 69 (1988) 545-558)) . Les animaux traités avec l'acide iboténique montrent un déficit des capacités d'apprentissage et de mémorisation. On observe également un déficit dans la localisation spatiale, similaire à celui observé dans la maladie d'Alzheimer. Pour les animaux traités les déficits sont nettement moindres et les capacités d'apprentissage et de mémorisation sont maintenues.

A l'issue des tests comportementaux, on sacrifie les animaux afin de procéder à une analyse histologique de la zone du noyau basalis. On observe une perte de substance induite par l'acide iboténique qui est nettement plus restreinte pour les animaux traités. La lésion qui pour les témoins s'étend dans toutes les directions à partir du site d'injection vers le globus pallidus est moins étendue après traitement et correspond à une injection de neurotoxine moins importante. Des dosages biochimiques sont également effectués sur le néocortex et l'hippocampe. L'activité de la choline acétyl transférase est diminuée dans le cortex frontal, temporal et pariétal, ainsi que dans l'amygdale deux semaines après l'injection d'acide iboténique. Le traitement avec les dérivés de l'invention entraîne une réduction sensible de ce déficit dans ces régions et on observe une récupération progressive de l'activité choline acétyl transférase dans les semaines qui suivent l'injection.

Les dérivés de l'invention permettent de protéger certains neurones cérébraux contre des lésions induites par des acides aminés excitotoxiques et contre les conséquence fonctionnelles de ces lésions.

3) Réparation de lésions

La régénérescence celulaire intervient également au cours de la réparation de lésions vasculaires ou cutanées. Ainsi l'endothélium vasculaire est composé d'une monocouche de cellules qui encadrent la face interne du sytème cardio-vasculaire. Cette monocouche représente une surface de plusieurs centaines de $m^2$ et joue un rôle majeur dans l'intégrité vasculaire. Les cellules endothéliales produisent de nombreuses substances qui participent à la coagulation, à la fibrinolyse et aux phénomènes inflammatoires. L'altération de ces fonctions peut conduire à une augmentation de la perméabilité vasculaire et à une accumulation de protéines et lipoprotéines dans le tissu intersticiel, ou à l'adhésion de plaquettes au sous-endothélium accompagnée de sécrétion de "platelet derived growth factors" (PDGF), ou enfin à la migration et la prolifération de cellules musculaires lisses. Une altération de l'endothélium vasculaire est donc une étape initiale importante dans les phénomènes de thrombose et d'athérosclérose (Cazenave et al. Inter Angio 3:27-32 (1984)). A la suite d'une lésion de l'endothélium vasculaire, plusieurs phénomènes apparaissent dans le processus de réparation in vivo: tout d'abord la migration et la réplication de cellules endothéliales, l'adhésion plaquettaire sur le sous-endothélium exposé et enfin la prolifération de cellules musculaires lisses qui conduit à la formation de plaques et à l'athérosclérose. Des dérivés permettant de protéger la paroi vasculaire ou d'inhiber l'adhésion plaquettaire peuvent donc empêcher la formation de plaques et l'athérosclérose.

Une des applications de l'invention consiste à utiliser les dérivés décrits ci-dessus pour favoriser la réparation de lésions cutanées ou vasculaires. Les lésions des cellules endothéliales entraînent la formation de dépôts de protéines ou lipoprotéines dans le tissu intersticiel, ainsi que la prolifération de cellules musculaires lisses. Ces phénomènes

constituent l'étape initiale dans la formation de thrombose et de l'athérosclérose (R.Ross. Atherosclerosis 1:293-311 (1981)). Les dérivés de l'invention augmentent la différenciation des cellules du derme, de l'épiderme et des cellules endothéliales vasculaires. Comme nous le montrons dans l'Exemple 3, ces dérivés accélèrent la réparation d'une lésion mécanique de cellules endothéliales vasculaires. Cette propriété est démontrée à l'aide d'un modèle in vitro de lésion mécanique de cellules endothéliales humaines en culture. Les cultures sont obtenues à partir de veines ombilicales humaines et sont maintenues dans un milieur RPMI plus HEPES plus 30% de sérum humain ; la lésion est réalisée 3 jours après la confluence par l'application d'un disque de cellulose sur une couché confluente de cellules endothéliales (C.Klein-Soyer et al Thrombosis Haemostasis 56:232-235(1986)). La vitesse de régénération dépend de la taille de la lésion initiale et de la concentration en sérum. De plus l'addition au milieu de culture des dérivés de l'invention augmentent significativement la surface de la lésion initiale recouverte par l'endothélium qui régénère. Les doses utilisées sont de l'ordre de 0,01 à 100µM, plus particulièrement 0,1 à 10µM. Les doses correspondantes pour l'utilisation in vivo sont de l'ordre de 0,01mg/kg à 100mg/kg, plus particulièrement 0,1 à 10mg/kg.

L'application des dérivés de la présente invention permet d'accélérer la réparation de lésions vasculaires ou cutanées et de retarder la dégénérescence des cellules de la peau, liée en particulier au vieillissement, à l'exposition aux rayons U.V et à des agressions chimiques. Cette propriété est démontrée in vitro à l'aide de cultures de kératinocytes. Les alcools gras à longue chaîne de l'invention augmentent la survie de ces cellules placées dans des conditions défavorables ou en présence de facteurs qui entraînent une dégénérescence de ces cellules. Ils accélèrent leur différenciation ainsi que la réparatietton de lésions tissulaires. Les doses utilisées sont similaires à celles décrites pour les lésions vasculaires.

**EXEMPLE 3 : REPARATION DE LESIONS DE L'ENDOTHELIUM VASCULAIRE PAR DES ALCOOLS GRAS A LONGUE CHAINE.**

Des modèles de lésion mécanique d'une couche de cellules endothéliales in vitro permettent d'étudier les processus de réparation de ce type de lésions (C.Klein-Soyer et al sus-mentionné). Les cellules endothéliales sont isolées à partir de veines ombilicales humaines. On les incube avec de la collagénase 0,1% pendant 15 min à 37°C et on récupère l'effluent dans des tubes contenant du milieu de culture (RPMI, HEPES, glutamine et 5 à 30% de sérum humain). Après centrifugation on ensemence le culot dans des boites de Petri prétraitées avec de la fibronectine qui sert de support.

La lésion se fait sur des cultures confluentes par application d'un disque calibré de polyacetate de cellulose. Cette technique détache sélectivement les cellules endothéliales qui se trouvaient dans la zone d'application du disque. Puis on ajoute au milieu de culture les alcools gras à longue chaîne à des doses variant de 0,01 µM à 100 µM, et préférentiellement 0,1 à 10 µH.

Après la lésion on retire des boites toutes les 24h afin de mesurer la taille de la lésion. On utilise des grilles adhésives divisées en carrés de 0,5mm$^2$ qu'on applique sur le fond de la boite, en coincidence avec la lésion. On mesure la surface de la lésion en comptant les carrés avec un microscope inversé. La vitesse de régénération est linéaire pendant 48h, puis se ralentit quand la lésion n'est plus que de 75 à 80% de la lésion initiale. La présence des dérivés de l'invention augmente la vitesse de régénérescence aux temps précoces et même en absence de sérum humain dans le milieu de culture. De plus en l'absence de sérum on observe un début de dégénérescence des cellules endothéliales, ce qui n'est pas le cas en présence d'alcools gras à longue chaîne.

In vivo une lésion de l'endothélium vasculaire peut entraîner l'adhésion de plaquettes, la prolifération de cellules musculaires lisses et la formation de thrombose ou de plaques d'athérosclérose. A l'aide du modèle de lésion mécanique, on observe que les alcools gras à longue chaîne de l'invention protègent la paroi vasculaire et améliorent les capacités de régénérescence de celle-ci. Les propriétés physico-chimiques de ces dérivés et leur bonne biodisponibilité en font des outils thérapeutiques particulièrement utiles dans le traitement et la prévention de la thrombose et de l'athérosclérose.

4) <u>Cytoprotection</u>

Certaines lésions cellulaires sont liées à la présence de toxiques ou sont la conséquence d'une ischémie, comme dans le tissu nerveux. Ces phénomènes sont particulièrement importants dans le foie, le muscle cardiaque et le rein. Un certain nombre de molécules (xénobiotiques) peuvent altérer les fonctions des cellules hépatiques soit directement soit après conversion de ces molécules sous forme plus toxique. La lésion cellulaire peut débuter soit par la formation d'un complexe stable avec une protéine ou un autre composé intra-cellulaire, soit par la formation d'une entité chimique très réactive ou en induisant des changements physico-chimiques dans la cellule ou dans son environnement (Bridges et al Ann N. Y. Acad.Sci.407:42-63 (1983)). La lésion cellulaire peut être limitée ou empêchée par certains systèmes de défense, comme les enzymes du métabolisme des médicaments, des proteines de liaison ou des antioxydants. On peut utiliser des hépatocytes isolés comme modèle expérimental pour étudier la toxicité de certaines molécules. On mesure la cytotoxicité par la libération d'enzymes cytosoliques, la sécrétion d'albumine, des changements morphologiques observés en microscopie optique et par la détermination de la viabilité cellulaire. La lésion cellulaire est influencée par son environnement, en particulier la pression partielle d'oxygène ou la présence de calcium extra-cellulaire. La toxicité de certaines drogues est potentialisée par la déplétion en glutathion. L'utilisation d'hépatocytes isolés maintenus en culture permet d'étudier la cytotoxicité hépatique et également de mettre en évidence des composés protecteurs vis-à-vis des cytotoxiques. On peut étudier en particulier le maintien des cytochromes P450 et des enzymes du métabolisme des médicaments, comme la glucuronyltransferase et la sulfotransférase.

De même on peut étudier la cytotoxicité de certaines molécules sur les cellules du glomérule ou du tubule rénal à l'aide de cellules mésengiales, épithéliales ou tubulaires isolées. On mesure la cytotoxicité par des changements morphologiques ou par l'expression de fonctions biochimiques propres à ces cellules (sécrétion d'un inhibiteur d'activateur du plasminogène, de PGE2, d'interleukine 1) (J. D. Sraer et Coll., FEBS Lett.104:420-424(1979)).

En particulier, les alcools gras à longue chaîne de l'invention, augmentent la synthèse et la secrétion des activateurs du Plasminogène de l'ordre de 70 à 100% par rapport au contrôle, dans des cellules mésengiales et épithéliales de rein humain. L'effet maximum est obtenu à des doses allant de $10^{-5}$ à $10^{-7}$ M. Ce résultat permet d'envisager une action pharmacologique dans les phénomènes de coagulation et dans la formation des matrices extra-cellulaires. Les molécules de l'invention peuvent également empêcher la propagation de métastases provenant de tumeurs cancéreuses, et s'opposer en cela à l'action de certaines protéines.

L'augmentation de la synthèse de tPA (activateur tissulaire de plasminogène) par les cellules mésangiales en présence de n-hexacosanol est représentée sur la Figure 2.

En ce qui concerne le muscle cardiaque, les lésions cellulaires sont généralement la conséquence d'une ischémie. Des cellules musculaires cardiaques isolées permettent en particulier de suivre l'évolution de lésions sur le plan des modifications électrophysiologiques (P. Athias et al. In Vitro 22:44(1986)).

Les dérivés d'alcools gras à longue chaîne de l'invention jouent un rôle dans la protection contre des lésions chimiques ou liées à une ischémie qui s'exercent sur des cellules hépatiques, rénales ou musculaires cardiaques.

La présence de composés toxiques ou une ischémie provoque une dégénérescence cellulaire qui se manifeste par une diffusion d'enzymes cytosoliques (LDH), des changements morphologiques, une perte de la viabilité cellulaire. On peut utiliser des cellules en culture pour mettre en évidence cette toxicité et d'éventuels agents protecteurs. Comme nous le démontrons dans l'Exemple 4, les dérivés de l'invention augmentent la survie et maintiennent la différenciation d'hépatocytes maintenus en culture. Les cultures sont obtenues par fractionnement à partir de foie de rats adultes et sont maintenues dans un milieu Ham/F12 additionné d'albumine, insuline, hydrocortisone et 10% de sérum de veau foetal. La survie des hépatocytes in vitro est limitée, ils ont tendance à perdre certains caractères de différenciation et à dégénérer en présence de composés toxiques. C'est le cas en particulier pour la galactosamine, le paracétamol, le pentobarbital (Paine et Hockin. Toxicology 25:41(1982) ou le cyclophosphamide (ACOSTA et MITCHEL. Bioch. Pharm. 30:3225(1981).

L'addition d'alcools gras à longue chaîne dans le milieu de culture améliore la survie des hépatocytes in vitro, comme on peut le mesurer par des tests de viabilité, par le maintien du glutathion et de l'activité cytochrome P450, ainsi que la sécrétion d'albumine (Le Rumeur et al. Exp.Cell Res.147:247-254(1983)). Ces dérivés de l'invention diminuent également la toxicité du pentobarbital ou du cyclophosphamide, comme on peut l'observer par les changements morphologiques, la diffusion d'enzymes cytosoliques et l'activité d'enzymes de détoxification, comme les cytochromes P450, les monooxygénases et les enzymes de conjugaison. Les doses utilisées sont de l'ordre de 0,01 à 100µM, plus particulièrement 0,1 à 10µM. Les doses correspondantes pour l'utilisation in vivo sont de l'ordre de 0,01mg/kg à 100mg/kg, plus particulièrement 0,1 à 10mg/kg.

L'application des dérivés de la présente invention permet de protéger les cellules hépatiques, rénales ou musculaires cardiaques contre la dégénérescence due à des composés toxiques ou à une ischémie. L'effet protecteur pour des cellules du glomérule rénal est également démontré à l'aide de cellules mésengiales, épithéliales ou tubulaires in vitro. On peut mesurer l'effet sur la dégénérescence cellulaire par des changements morphologiques et par l'expression de fonctions biochimiques spécifiques, comme la sécrétion d'inhibiteur d'activateur du plasminogène, de PGE2, d'interleukine 1 (J. D. Sraer et al. sus-mentionné). On peut mesurer l'effet sur la dégénérescence de cellules musculaires cardiaques, en particulier après une ischémie, par des changements morphologiques, la diffusion d'enzymes cytosoliques(LDH) ou des modifications des propriétés excitatrices membranaires (P. Athias et al. sus-mentionné). Les doses utilisées sont similaires à celles décrites pour l'effet cytoprotecteur des hépatocytes.

Les dérivés de la présente invention permettent de protéger les cellules intestinales et gastriques vis-à-vis de composés toxiques, comme un excès d'acidité gastrique. Les molécules de l'invention rendent les cellules pariétales et épithéliales de l'estomac plus résistantes vis-à-vis d'un excès d'acidité gastrique ; elles diminuent la sécrétion des ions $H^+$ et augmentent la secrétion de mucus. Cet effet permet de protéger cet organe contre les conséquences d'un stress, de régulariser la motricité intestinale dans une situation de stress et d'empêcher la formation d'un ulcère gastrique ou intestinal. Les dérivés de l'invention augmentent également la trophicité des villosités intestinales, des cellules épithéliales de l'estomac et des ilots de Langerhans du pancréas. Ils rendent la muqueuse intestinale plus résistante en situation de malnutrition et limitent les conséquences fonctionnelles liées à une pancréatite, en particulier sur la sécrétion

d'insuline. Les dérivés de l'invention sont ajoutés avantageusement à des préparations destinées à l'alimentation parentérale ou à des substituts du lait maternel. Ils favorisent la différenciation et la maturation des tissus du tractus digestif au cours du développement ou à la suite d'un traumatisme chimique, mécanique ou chirurgical.

**EXEMPLE 4 : PROTECTION DES CELLULES HEPATIQUES VIS-A-VIS DE LA DEGENERESCENCE INDUITE PAR DES COMPOSES TOXIQUES.**

Le maintien en culture d'hépatocytes isolés permet d'étudier leur métabolisme et leur sensibilité vis-à-vis de certains composés toxiques. Les hépatocytes sont isolés à partir de foie de rats adultes par perfusion avec de la collagénase, filtration et centrifugation dans du milieu L15. On purifie les hépatocytes par élutriation dans une chambre d'élutriation à un débit de 10 à 40 ml/min qui est centrifugée à 840 tours par min (E.Le Rumeur et al sus-mentionné). Une fraction pure contenant des hépatocytes diploides est obtenue à un débit de 15ml/min. On les ensemence dans des boites de Pétri couvertes d'une couche de fibronectine, dans du milieu Ham F12 contenant de l'albumine bovine, de l'insuline, 10% de sérum foetal de veau et de l'hydrocortisone. On ajoute dans certaines boites des alcools gras à longue chaîne à des doses variant de 0,01μM à 10μM, et préférentiellement 0,1 à 10μM.

On peut mesurer la différenciation des hépatocytes en mesurant la production d'albumine par immunonéphélométrie et la synthèse protéique par incorporation de leucine marquée. On peut également mesurer le taux de glutathion et l'activité d'enzymes du métabolisme des médicaments comme le cytochrome P450, les glucuronyltransférases et sulfotransférases. Les cellules ont tendance à dégénérer au cours de la culture et perdent en particulier leur activité cytochrome P450 et les enzymes de conjugaison. Par contre les cultures traitées par les molécules que nous décrivons ont un meilleur taux de survie et conservent leurs activités enzymatiques. Ces résultats montrent l'effet cytotrophique de ces molécules.

L'addition de certaines drogues pendant 24h à des doses de 0,1 à 1mM, comme la galactosamine, le paracétamol, le pentobarbital ou le cyclophosphamide entraîne une dégénérescence des hépatocytes, qu'on observe par des changements morphologiques et biochimiques. Les cellules libèrent des enzymes cytosoliques dans le milieu de culture (comme la LDH), la viabilité cellulaire diminue, ainsi que la capacité de sécrétion d'albumine et le taux de glutathion. La dégénérescence est sensiblement atténuée dans les cultures traitées par des alcools gras à longue chaîne, selon l'invention, avec amélioration de la survie et maintien des caractères de différenciation des hépatocytes.

Ces résultats montrent l'intérêt des dérivés de l'invention dans le traitement de la toxicité induite par certaines drogues et comme protecteurs de la dégénérescence des cellules hépatiques.

5) Défenses immunitaires

Un défaut d'expression des fonctions différenciées de certaines cellules peut avoir pour conséquence un déficit dans les défenses immunitaires de l'organisme. Celles-ci reposent en particulier sur les macrophages qui sécrètent des cytokines, qui participent à la régulation de l'immunité. L'interleukine 1 est sécrétée par les macrophages ; elle favorise la prolifération et la différenciation des lymphocytes, en augmentant l'apparition de récepteurs à l'IL2 (Hogan et Vogel. J. Imunol.Immunophamacol.8:6-15(1988)). Elle favorise également la différenciation des lymphocytes B et la sécrétion d'anticorps. Les macrophages sécrètent également une molécule appelée "Tumor Necrosis Factor"(TNF) qui entraîne la lyse de tumeurs et une autre appelée "Colony Stimulating Factor"(CSF) qui peut favoriser la production d'interféron dans la moelle. Ce CSF est lui-même un agent de différenciation pour les précurseurs des macrophages ; il augmente la réponse des macrophages à certains agents de différenciation comme l'ester de phorbol. Enfin les macrophages produisent l'interféron $\alpha$ et $\beta$ en réponse à certains stimuli. L'interféron augmente les fonctions phagocytaires des macrophages et donc leur rôle tumoricide.

Les défenses immunitaires reposent également sur les lymphocytes. Ceux-ci peuvent se différencier en monocytes ou en granulocytes, en réponse à certains agents. Ainsi un diester de phorbol favorise la différenciation de lymphocytes en monocytes (Rovera et al. Proc.Natl.Acad.Sci. 76:2779(1979)) et les rétinoides favorisent la différenciation des lymphocytes en granulocytes (Breitman et al. Proc.Natl.Acad.Sci. 77:2939(1980)).

Une autre application intéressante de la présente invention consiste à favoriser la différenciation des cellules qui participent aux défenses immunitaires de l'organisme, comme les macrophages, les monocytes et les lymphocytes. On peut mesurer le degré de différenciation de ces cellules par leur capacité de sécrétion de certaines cytokines, comme l'IL1 (interleukine-1), le CSF ou l'interféron, ainsi que leur réponse aux signaux immunitaires (Waren et Ralph. J.Immunol, 137:2281(1986)). L'addition d'alcools gras à longue chaîne de l'invention a un effet cytotrophique sur ces cellules et augmente la production de CSA (Colony Stimulating Activity) par les monocytes en réponse au PMA, augmente la production d'interféron et d'interleukine 1 par les macrophages, augmente leurs fonctions phagocytaires, ainsi que leur réceptivité aux signaux immunitaires. les dérivés de l'invention favorisent également la prolifération et la différenciation des macrophages péritonéaux et cérébraux; ils augmentent ainsi leurs capacités cytotoxiques et phagocytaires, permettant d'accroître les défenses de l'organisme au cours d'une infection bactérienne ou virale (SIDA en particulier). La

capacité d'agir au niveau du SNC, les rend particulièrement intéressants en cas d'atteinte cérébrale par les virus (HIV en particulier). Ces composés augmentent également l'activité LAK (Lymphokine activated killer) des lymphocytes T, ainsi que l'activité cytotoxique de certains lymphocytes T (ils stimulent la sécrétion d'IL-2 chez le lymphocyte). Si l'on incube des monocytes sanguins en présence des dérivés de l'invention, on observe une amélioration de leur survie après 5 jours, une meilleure différenciation en qualité et en nombre de cellules différenciées (apparition d'un antigène de surface spécifique, sécrétion de certaines cytokines). Une production accrue d'ILI favorise l'activation des lymphocytes B et T, augmente le nombre de récepteur à l'IL2, induit une granulocytose. Les doses utilisées in vitro sont de l'ordre de 0,01 à 10µM, plus particulièrement 0,1 à 5µM. Les doses correspondantes pour l'utilisation in vivo sont de l'ordre de 0,01mg/kg à 10mg/kg, plus particulièrement 0,1 à 5mg/kg.

L'action trophique des dérivés de l'invention sur les lymphocytes et les macrophages, leur permet d'être utilisés pour favoriser les défenses immunitaires de l'organisme, en particulier dans le cas de déficit du système immunitaire (SIDA, maladies auto-immmunes).

6) Effet anti-inflammatoire et anti-allergique

L'histamine joue un rôle primordial dans la pathologie de l'inflammation ; c'est aussi un des médiateurs des phénomènes allergiques ou anaphylactiques. Elle est libérée par les mastocytes ou les leucocytes basophiles. La libération d'histamine par les mastocytes pourrait également jouer un rôle dans le contrôle de la microcirculation cutanée. En dehors de ce phénomène non immunologique, il est reconnu que la stimulation des basophiles et des mastocytes par un antigène et des IgE est un mécanisme fondamental dans des pathologies allergiques aigües comme le choc anaphylactique, l'asthme, la rhinite allergique et certaines affections de la peau, comme l'urticaire et la dermatite atopique (J. C. Foreman Ann. Rev. Pharmacol. Toxicol. 21: 63-81(1981)).

Les anticorps IgE sont sécrétés par des lymphocytes B en réponse à la fixation d'un antigène. Cette sécrétion est contrôlée par d'autres lymphocytes, les lymphocytes T suppresseurs et les lymphocytes T helpers. L'IgE se lie ensuite aux mastocytes et aux basophiles et entraîne la libération d'histamine plus d'autres médiateurs de l'inflammation. Le crosslinking des IgE est nécessaire afin d'obtenir cette réponse et la quantité d'histamine libérée est liée au nombre de molécules IgE crosslinkées. La libération d'histamine nécessite aussi la présence de calcium extra-cellulaire, ainsi que l'activation du métabolisme des inositols phosphates et l'intervention de nucléotides cycliques. Un certain nombre de composés qui interagissent soit sur le flux de calcium, soit sur le métabolisme des inositols phosphates, soit sur celui des nucléotides cycliques, comme la théophylline, modifient la réponse des mastocytes et des basophiles à la fixation des IgE.

Enfin la réponse physiologique à la sécrétion d'histamine et des autres médiateurs de l'inflammation (leucotriènes, prostaglandines) passe par la fixation de ces molécules à leur cible, en particulier les muscles lisses. Citons en exemple la vasoconstriction bronchique, consécutive à la libération d'histamine et qui intervient dans la maladie asthmatique.

Les dérivés d'alcools gras à longue chaîne de l'invention présentent un effet cytotrophique sur des cellules qui participent aux phénomènes inflammatoires et allergiques de l'organisme, comme les mastocytes et les leucocytes basophiles. Les phénomènes inflammatoires et allergiques sont déclenchés par la fixation d'un antigène et des IgE sur ces cellules, ce qui entraîne la libération d'histamine. On peut mesurer l'histamine libérée par des mastocytes provenant du liquide péritonéal en les incubant pendant 30 min à 37°C dans un milieu défini. La quantité d'histamine secrétée augmente avec la concentration en calcium dans ce milieu ou en présence de l'ionophore A23187 ; l'histamine est également secrétée à la suite de la fixation d'IgE sur la membrane cellulaire et du crosslinking de ces molécules (J.C.Foreman sus-mentionné). Si l'on ajoute des dérivés de l'invention dans le milieu d'incubation, la quantité d'histamine libérée est significativement diminuée. Ceci suggère qu'ils agissent sur les flux de calcium ou sur le mouvement membranaire des récepteurs aux IgE, ou encore sur les mécanismes secondaires impliqués dans le processus d'exocytose (métabolisme des inositols phosphates, des nucléotides cycliques), avec pour conséquence une diminution de la sécrétion d'histamine et donc un effet anti-inflammatoire. Cet effet est obtenu in vitro avec des quantités d'alcools gras à longue chaîne de l'ordre de 0,01 à 100 µM, plus particulièrement 0,1 à 10µM. Les doses correspondantes pour l'utilisation in vivo sont de l'ordre de 0,01mg/kg à 100 mg/kg, plus particulièrement 0,1 à 10 mg/kg.

Les dérivés de l'invention permettent donc d'atténuer les processus inflammatoires et allergiques aigus, consécutifs à la sécrétion d'IgE et à la fixation d'un antigène sur les mastocytes et les basophiles, comme on peut les observer dans la maladie asthmatique, la rhinite allergique, le choc anaphylactique ou l'urticaire.

Les dérivés de l'invention protègent le tissu cartilagineux et osseux des conséquences d'une inflammation aigüe ou chronique, comme on peut les observer dans toute inflammation des articulations et des os. Ils ralentissent en particulier la destruction osseuse et la formation d'arthrose. Cet effet est obtenu grâce à un effet trophique des alcools gras à longue chaîne sur les chondrocytes et les ostéoblastes, ainsi qu'à une action favorisante sur la différenciation de ces cellules au cours du développement ou à la suite d'un traumatisme.

7) Effet anti-tumoral

Chaque cellule peut être identifiée par des critères morphologiques, biochimiques et immunologiques représentatifs d'un état donné qui peuvent changer sous l'effet d'une activation. Les phénomènes d'activation apparaissent rapidement après la stimulation : ce sont des variations de mobilisation calcique, du contenu en ADN, ARN ou en protéines ; elles aboutissent à l'expression d'antigènes membranaires ou à l'induction d'activités enzymatiques. Dans le cas où la stimulation conduit à l'acquisition de caractères spécifiques, antigéniques ou fonctionnels, correspondant à un état cellulaire plus mature que l'état initial, le signal activateur est un signal différenciateur. Ces effets sont observés avec divers types cellulaires, comme des neurones centraux avec l'hexacosanol (Borg et al. Neurosci.Lett.213:406-410(1987)), mais aussi des promyélocytes humains qui se différencient en monocytes en présence de diester de phorbol (G.Rovera et al Proc.Natl.Acad.Sci. 76:2779-2783(1979)) ou en granulocytes sous l'action des rétinoides (T.R.Breitman et al. Proc.Natl.Acad.Sci. 77:2936-2940(1980)).

Dans le cas des cellules promyélocytaires, la voie dans laquelle s'oriente la différenciation peut être précisée avec des marqueurs phénotypiques, comme l'expression d'antigènes membranaires MO2, spécifique des monocytes, ou MO1, caractéristique de la différenciation en granulocytes ou monocytes. Certains inducteurs ont la capacité de différencier des cellules cancéreuses ou précancéreuses en cellules différenciées. D'autres molécules comme le TNF ont également une activité anti-cancéreuse. Ce TNF est sécrété par les macrophages ; il peut provoquer la lyse de tumeurs. L'activité cytotoxique est exercée par des monocytes activés par le TNF (E.A.Carswell et al Proc.Natl.Acad.Sci. 25:3666 (1975)). Cette toxicité est sélective pour des cellules tumorales et on peut penser que cette substance joue un rôle primordial dans la défense de l'organisme contre la néoplasie. Toute molécule qui favoriserait la différenciation des macrophages ou des monocytes et leur production de TNF pourrait donc augmenter les capacités de défense de l'organisme.

Une autre application de l'invention consiste à favoriser la différenciation des cellules qui participent à la défense de l'organisme contre les processus tumoraux, comme les macrophages, les lymphocytes et les monocytes. Lorsqu'elles atteignent leur stade différencié, ces cellules montrent une activité phagocytaire et la libération de molécules tumoricides, comme le TNF. Les cellules promyélocytaires peuvent se différencier en monocytes ou en granulocytes sous l'effet de certains inducteurs comme les esters de phorbol ou les rétinoides. On peut mesurer la différenciation de ces cellules à l'aide de cellules promyélocytaires leucémiques HL60 maintenues en culture, dans un milieu RPMI contenant 20% de sérum de veau foetal. Les cellules sont mises en culture à $3x10^5$ cellules par ml et repiquées deux fois par semaine (N.Mendelsohn et al. Cancer Res. 40:1469-1474 (1980)).

On ajoute les dérivés de l'invention dans le milieu au moment de la mise en culture et on mesure le degré de différenciation 5 jours après le début du traitement par les changements de morphologie et par leur capcité de réduire le nitrobleu de tétrazolium, comme cela est montré dans l'Exemple 5. On observe une augmentation du nombre de cellules différenciées par rapport au contrôle dans les cultures traitées. De même les cellules traitées montrent une diminution du nombre de récepteurs à la transferrine, observée par immunofluorescence directe et un plus grand nombre d'antigènes membranaires MO1 observés par immunofluorescence indirecte. Les doses d'alcools gras à longue chaîne de l'invention utilisées sont de l'ordre de 0,01 à 100µM en culture, plus particulièrement 0,1 à 10µM. Les doses correspondantes pour l'utilisation in vivo sont de l'ordre de 0,1 à 10mg/kg.

L'utilisation des dérivés de l'invention permet donc de favoriser la différenciation des cellules cancéreuses ou précancéreuses, en particulier les précurseurs des macrophages, lymphocytes et monocytes. Ils augmentent en particulier leur activité phagocytaire et tumoricide.

**EXEMPLE 5 : EFFET CYTOTROPHIQUE SUR DES PROMYELOCYTES LEUCEMIQUES ML60**.

L'utilisation de promyélocytes leucémiques HL60 permet d'étudier in vitro leur différenciation en monocytes ou en granulocytes. Les cellules HL60 sont cultivées en milieu RPMI contenant 20% de sérum foetal de veau ; elles sont ensemencées à une densité de $3x10^5$ cellules par ml et repiquées deux fois par semaine (N.Mendelsohn et al sus-mentionné). Au moment de la mise en culture on ajoute des alcools gras à longue chaîne de l'invention à des concentrations allant de 0,01 à 100µM qu'on utilise comme signal différenciateur. Après 5 jours de culture, on étudie la différenciation des cellules HL60 en monocytes ou en granulocytes, qu'on peut mesurer par des changements morphologiques après coloration au May-Grünwald-Giemsa et par leur capacité de réduire le nitrobleu de tétrazolium. Les cellules indifférenciées contiennent moins de 3% de cellules capables de réduire ce composé. Dans les cultures traitées par les dérivés de l'invention, ce pourcentage est significativement augmenté, alors que le volume cellulaire diminue sensiblement. On observe également une alcalinisation du milieu intra-cellulaire, caractéristique de la différenciation en monocytes ou en granulocytes.

Pour mesurer l'expression des récepteurs à la transferrine, on utilise une méthode d'immunofluorescence directe ; après avoir rincé les cellules, on les incube avec l'anticorps antirécepteur à la transferrine couplé à la fluorescéine pendant 1 h à +4°C. On filtre la suspension cellulaire sur voile de nylon, avant le passage au cytofluoromètre. On observe

une diminution des récepteurs à la transferrine dès le 2° jour de culture. On étudie ensuite l'expression de marqueurs phénotypiques, comme les antigènes membranaires M02, spécifiques des monocytes, et MO1, présents sur les monocytes et les granulocytes. Le marquage se fait avec des anticorps anti-MO1 et anti-MO2 (Coultronics), suivi d'une incubation pendant 1 h à +4°C avec l'anticorps de lapin anti-souris couplé à la fluorescéine. A partir de 3 jours de culture, le pourcentage de cellules exprimant MO1 augmente, alors que l'expression de MO2, spécifique des monocytes apparaît après 4 ou 5 jours, lorsque les cultures sont maintenues en présence d'alcools gras à longue chaîne de l'invention.

Le marquage à l'iodure de propidium montre que les cellules s'accumulent dans la phase G0/Gl du cycle cellulaire. Au cours de la différenciation de cellules HL60, on observe également une augmentation de l'activité protéine kinase AMPc dépendante, une augmentation de l'activité de la transglutaminase et une diminution de l'expression de l'oncogène c-myc.

Ces résultats montrent que les alcools gras à longue chaîne de l'invention utilisés à des doses allant de 0,01µM à 100µM, et plus particulièrement 0,1 à 10µM, permettent d'induire la différenciation de promyélocytes en granulocytes ou en monocytes. Les cellules induites possèdent les caractéristiques de différenciation des granulocytes et monocytes, en particulier leur activité phagocytaire, qui leur permet de jouer un rôle fondamental dans la protection de l'organisme contre le développement de tumeurs cancéreuses.

8) exemples de milieux de culture utilisés en association avec les dérivés de l'invention.

**a) Culture de cellules neuronales**

Minimum Essential Medium (Milieu Minimum Essentiel) modifié par DULBECCO (DMEM) selon DULBECCO, R., FREEMANN G., Virology, 8, (1959) 396, additionné de :

| transferrine | 100 µg/ml (concentrations finales) |
|---|---|
| insuline | 5 µg/ml |
| selenium | 30 nM |
| putrescine | 0,1 mM |
| progestérone | 20 nM |
| hexacosanol | 500 nM |

**b) Culture de kératinocytes ou de fibroblastes**

Minimum Essential Medium selon EAGLE, H., Science, 130, (1959), 432 additionné de :

| | |
|---|---|
| EGF (facteur de croissance de l'épiderme) | 10ng/ml |
| hydrocortisone | 0,4µg/ml |
| hexacosanol | 1 µM |

**c) Culture de cellules endothéliales ou mésengiales**

Milieu 199 selon MORGAN, J.F., et coll., Proc. Soc. Exp. Biol. Med.; 73 (1950), 1 :50 Vol%

Milieu RPMI 1640 selon MOORE, G.E. et coll., J. Am. Med. Assoc., 199, (1967) 519 :50 Vol% additionnés de :

| | |
|---|---|
| HEPES | 10 mM |
| L-glutamine | 2 mM |
| hexacosanol | 1 µM |

**d) Culture d'hépatocytes**

Milieu Ham F12 selon HAMM, R.G., Proc. Nat. Acad. Sci., 53 (1965) 288 additionné de :

| | |
|---|---|
| BSA (bovine serum albumine) | 1 g/l |
| insuline | 10 mg/l |
| hexacosanol | 10 μM |

**e) Culture de mycoplasmes**

Pour 200 ml de milieu complet, il faut :

- 140 ml de milieu PPLO, comprenant :

. protéines de coeur de boeuf 50 g
. peptone 10 g
. NaCl 5 g

(quantités pour préparer 1 l dans l'eau distillée)

- 60 ml d'une préparation comprenant :

. 25 % extrait de levure 1 part
. sérum de cheval normal 2 parts

- additionnés de :

| | |
|---|---|
| rouge de phénol à 0,4 % | 1.0 ml |
| glucose à 50 g pour 100 ml | 4.0 ml |
| pénicilline (100.000 μ./ml | 6.0 ml |
| acétate de thalium à 10 % | 0.5 ml |
| hexacosanol 20 mM | 0.5 ml |

**f) Culture de lymphocytes et macrophages**

Milieu RPMI 1640 selon MOORE et coll., J. Am. Med. Assoc., 199 (1967) 519 additionné de :

| | |
|---|---|
| L-alanine | 8,9 mg/l (concentration finale) |
| L-asparagine | 15,0 mg/l |
| acide L-aspartique | 13,3 mg/l |
| acide L-glutamique | 14,7 mg/l |
| glycine | 7,5 mg/l |
| L-proline | 11,5 mg/l |
| L-sérine | 10,5 mg/l |
| sérum de veau | 5 % |
| hexacosanol | 1μM |

Les légendes des Figures 1 et 2 qui suivent sont les suivantes :

- Figure 1 : augmentation de la survie neuronale en présence de dérivés du rétinal (ON13, ON9 et ON8); effet spécifique sur diverses zones cérébrales (cortex, septum, hippocampe).

- Figure 2 : augmentation de la synthèse de tPA (activateur tissulaire de plasminogène) par les cellules mésangiales en présence de n-hexacosanol.

## Revendications

**1.** Dérivés caractérisés par la formule suivante :

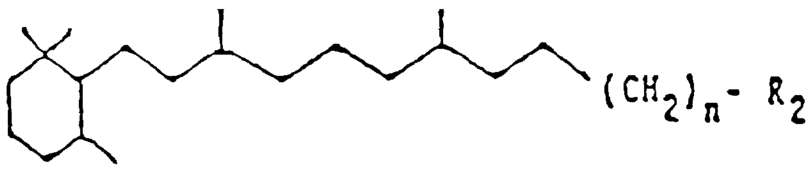

comportant, le cas échéant, 1 à 10, et de préférence 1 à 5, double(s) liaison(s) et dans laquelle $R_2$ représente :

(1) CN ou
(2) OR, R représentant un atome d'hydrogène, ou un groupe alcoyle de 1 à 3 atomes de carbone ou un groupe

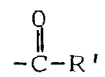
,

ou
(3)

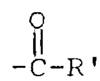
,

R' représentant, aux points (2) et (3) ci-dessus :

(a) un atome d'hydrogène,
(b) un groupe alcoyle de 1 à 5 atomes de carbone, de préférence de 1 à 3 atomes de carbone,
(c) un groupe de formule $OR_b$, $R_b$ représentant un groupe alcoyle de 1 à 3 atomes de carbone, ou
(d) un groupe amino de formule $-NR'_1R'_2$, dans laquelle $R'_1$ et $R'_2$ identiques ou différents représentent H ou un groupe alcoyle de 1 à 3 atomes de carbone et n est un nombre entier variant de 1 à 10.

**2.** Composition pharmaceutique comprenant au moins un dérivé selon la revendication 1 en association avec un véhicule physiologiquement acceptable.

**3.** Composition selon la revendication 2, comprenant entre 0,5 mg et 500 mg d'un dérivé selon l'une des revendications 1 à 2.

**4.** Un dérivé caractérisé par la formule :

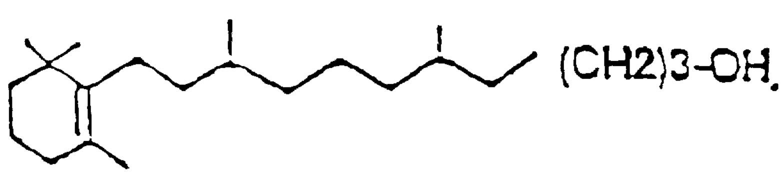

**5.** Un dérivé caractérisé par la formule :

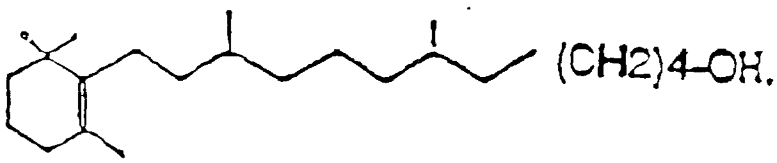

**6.** Un dérivé caractérisé par la formule :

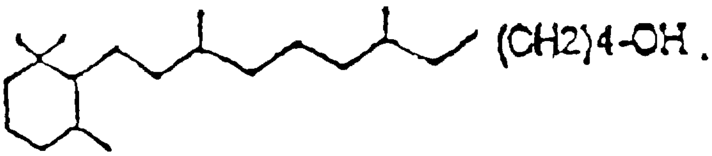

**7.** Un dérivé caractérisé par la formule :

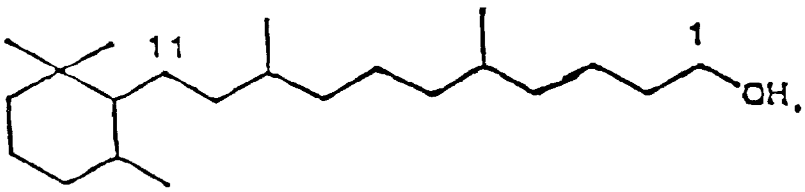

**8.** Un dérivé caractérisé par la formule :

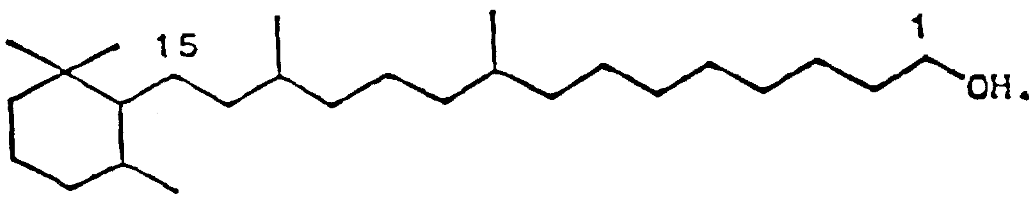

**9.** Un dérivé caractérisé par la formule :

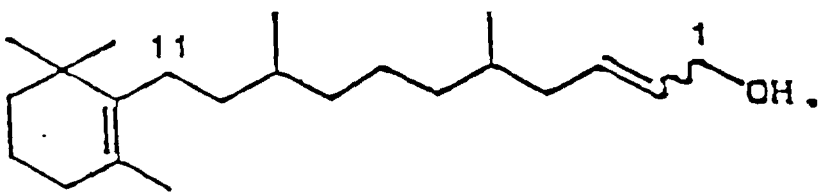

**10.** Utilisation d'un dérivé selon l'une quelconque des revendications 1 à 9 pour l'obtenir d'un medicament pour le traitement ou la prévention de désordres neuronaux.

**Patentansprüche**

**1.** Verbindungen, die durch die folgende Formel gekennzeichnet sind:

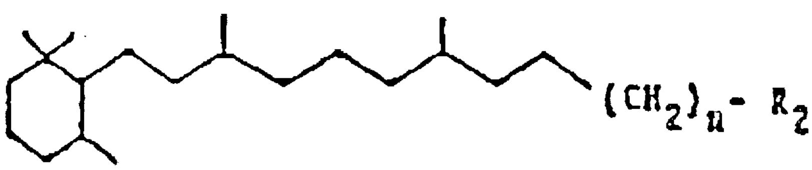

umfassend gegebenenfalls 1 bis 10 und vorzugsweise 1 bis 5 Doppelbindungen und worin $R_2$ angibt:

(1) CN oder

(2) OR, wobei R für ein Wasserstoffatom, eine Alkylgruppe mit 1 bis 3 Kohlenstoffatomen oder eine Gruppe steht, oder

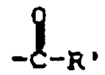

(3)

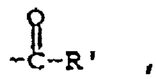

wobei R' zu Punkt (2) und (3) angibt:

(a) ein Wasserstoffatom,

(b) eine Alkylgruppe mit 1 bis 5 Kohlenstoffatomen, vorzugsweise 1 bis 3 Kohlenstoffatome,

(c) eine Gruppe der Formel $O_{Rb}$, wobei $R_b$ für eine Alkylgruppe mit 1 bis 3 Kohlenstoffatomen steht oder

(d) eine Aminogruppe der Formel $-NR'_1R'_2$, worin $R'_1$ und $R_2$ gleich oder verschieden sind und für H oder eine Alkylgruppe mit 1 bis 3 Kohlenstoffatomen stehen, und n ist eine ganze Zahl von 1 bis 10.

**2.** Pharmazeutische Zusammensetzung, die mindestens eine Verbindung nach Anspruch 1 zusammen mit einem physiologisch akzeptablen Trägermittel umfaßt.

**3.** Zusammensetzung nach Anspruch 2, umfassend 0,5 mg bis 500 mg einer Verbindung nach einem der Ansprüche 1 oder 2.

**4.** Verbindung, gekennzeichnet durch die Formel:

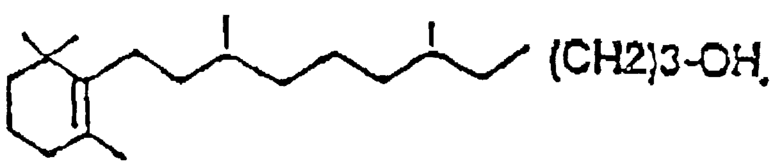

**5.** Verbindung, gekennzeichnet durch die Formel:

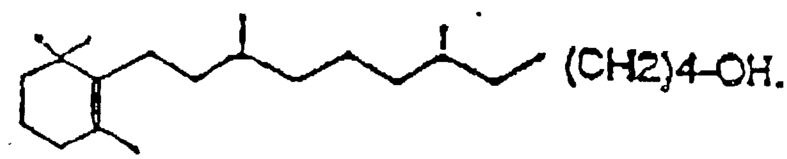

**6.** Verbindung, gekennzeichnet durch die Formel:

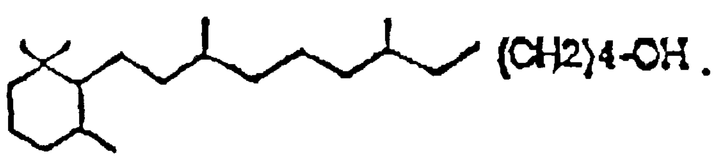

**7.** Verbindung, gekennzeichnet durch die Formel:

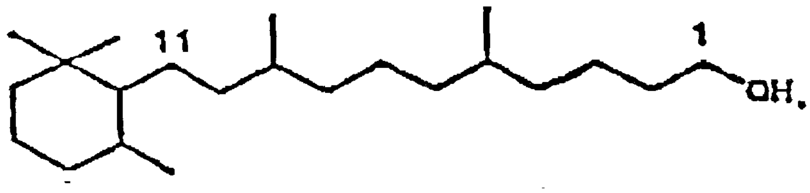

8. Verbindung, gekennzeichnet durch die Formel:

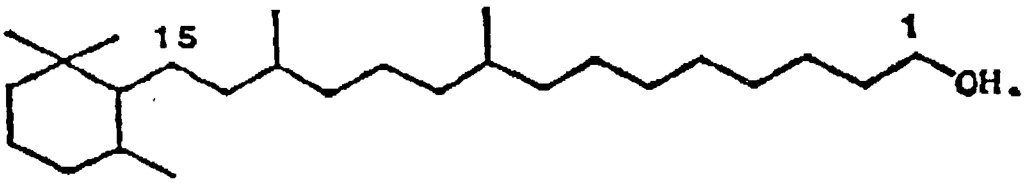

9. Verbindung, gekennzeichnet durch die Formel:

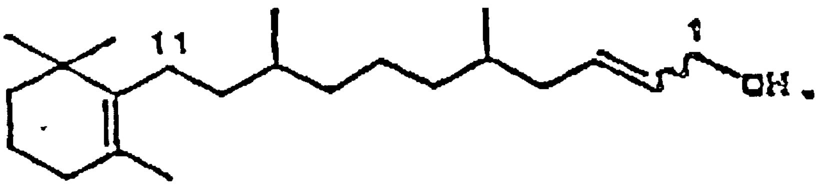

10. Verwendung einer Verbindung nach einem der Ansprüche 1 bis 9 zur Herstellung eines Medikaments zur Behandlung oder Prävention neuronaler Störungen.

## Claims

1. Derivatives characterised by the following formula:

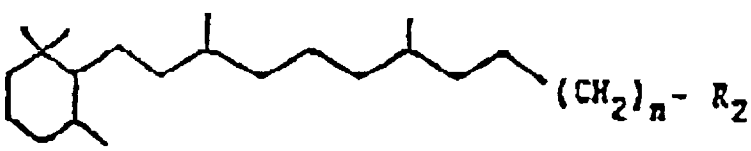

optionally comprising from 1 to 10, and preferably from 1 to 5, double bond(s) and in which
$R_2$ represents:

(1) CN or
(2) OR, R representing a hydrogen atom, or an alkyl group having from 1 to 3 carbon atoms or a group

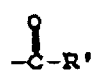 ,

or
(3)

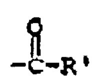 ,

R' representing, at points (2) and (3) above:

(a) a hydrogen atom,

(b) an alkyl group having from 1 to 5 carbon atoms, preferably from 1 to 3 carbon atoms,
(c) a group of the formula $OR_b$, $R_b$ representing an alkyl group having from 1 to 3 carbon atoms, or
(d) an amino group of the formula $-NR'_1R'_2$, in which $R'_1$ and $R'_2$, which may be identical or different, represent H or an alkyl group having from 1 to 3 carbon atoms and n is an integer from 1 to 10.

**2.** Pharmaceutical composition comprising at least one derivative according to Claim 1 in association with a physiologically acceptable carrier.

**3.** Composition according to Claim 2, comprising between 0.5 mg and 500 mg of a derivative according to either Claim 1 or Claim 2.

**4.** A derivative characterised by the formula:

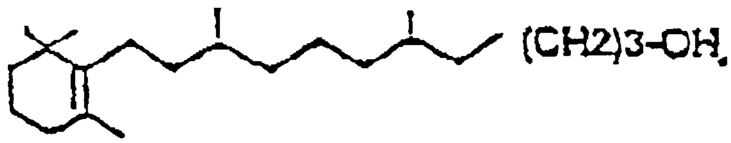

**5.** A derivative characterised by the formula:

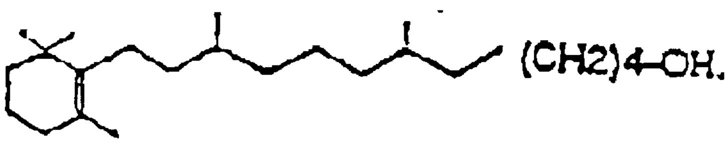

**6.** A derivative characterised by the formula:

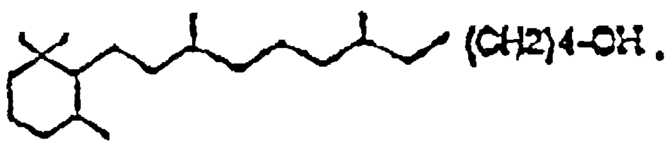

**7.** A derivative characterised by the formula:

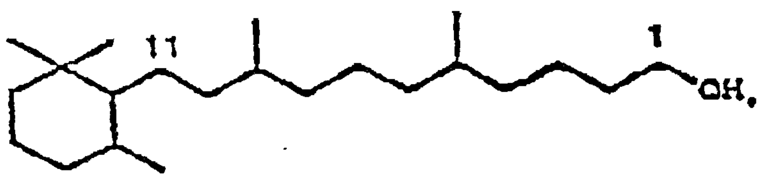

**8.** A derivative characterised by the formula:

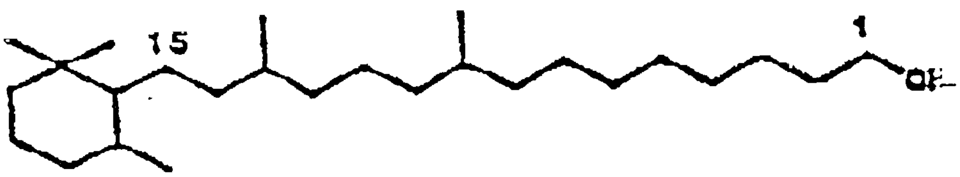

**9.** A derivative characterised by the formula:

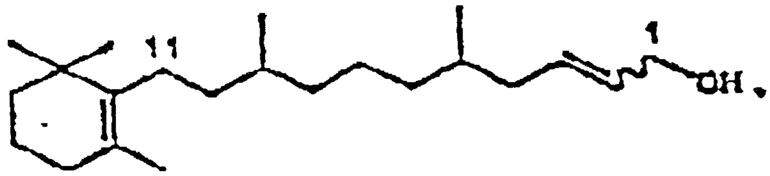

**10.** Use of a derivative according to any one of Claims 1 to 9 for obtaining a medicament for the treatment or prevention of neuronal disorders.

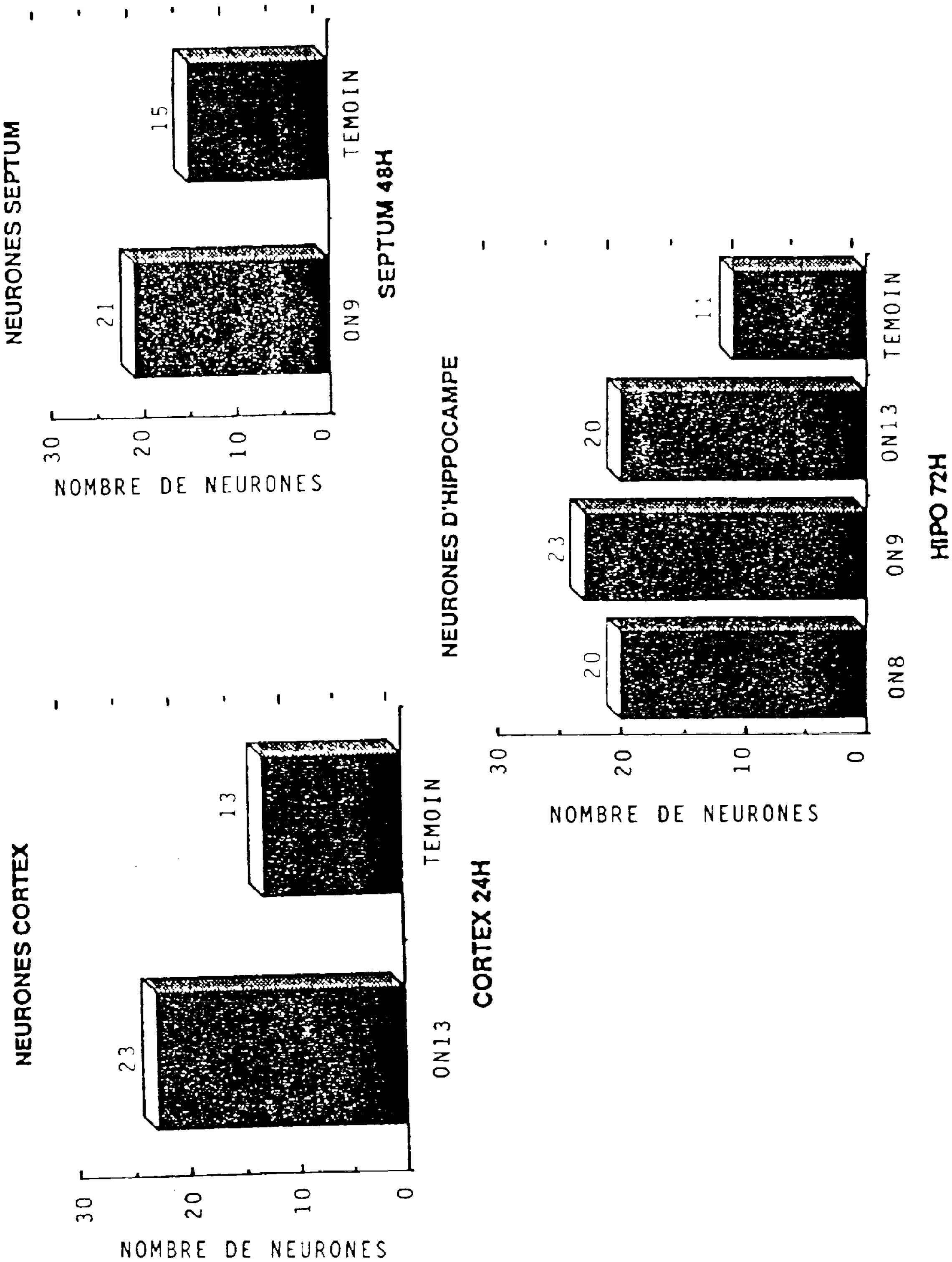
NEURONES SEPTUM
21
15
ON9
TEMOIN
SEPTUM 48H
30
20
10
0
NOMBRE DE NEURONES
NEURONES D'HIPPOCAMPE
20
23
20
ON8
ON9
ON13
TEMOIN
HIPO 72H
30
20
10
0
NOMBRE DE NEURONES
NEURONES CORTEX
23
13
ON13
TEMOIN
CORTEX 24H
30
20
10
0
NOMBRE DE NEURONES

FIG. 1

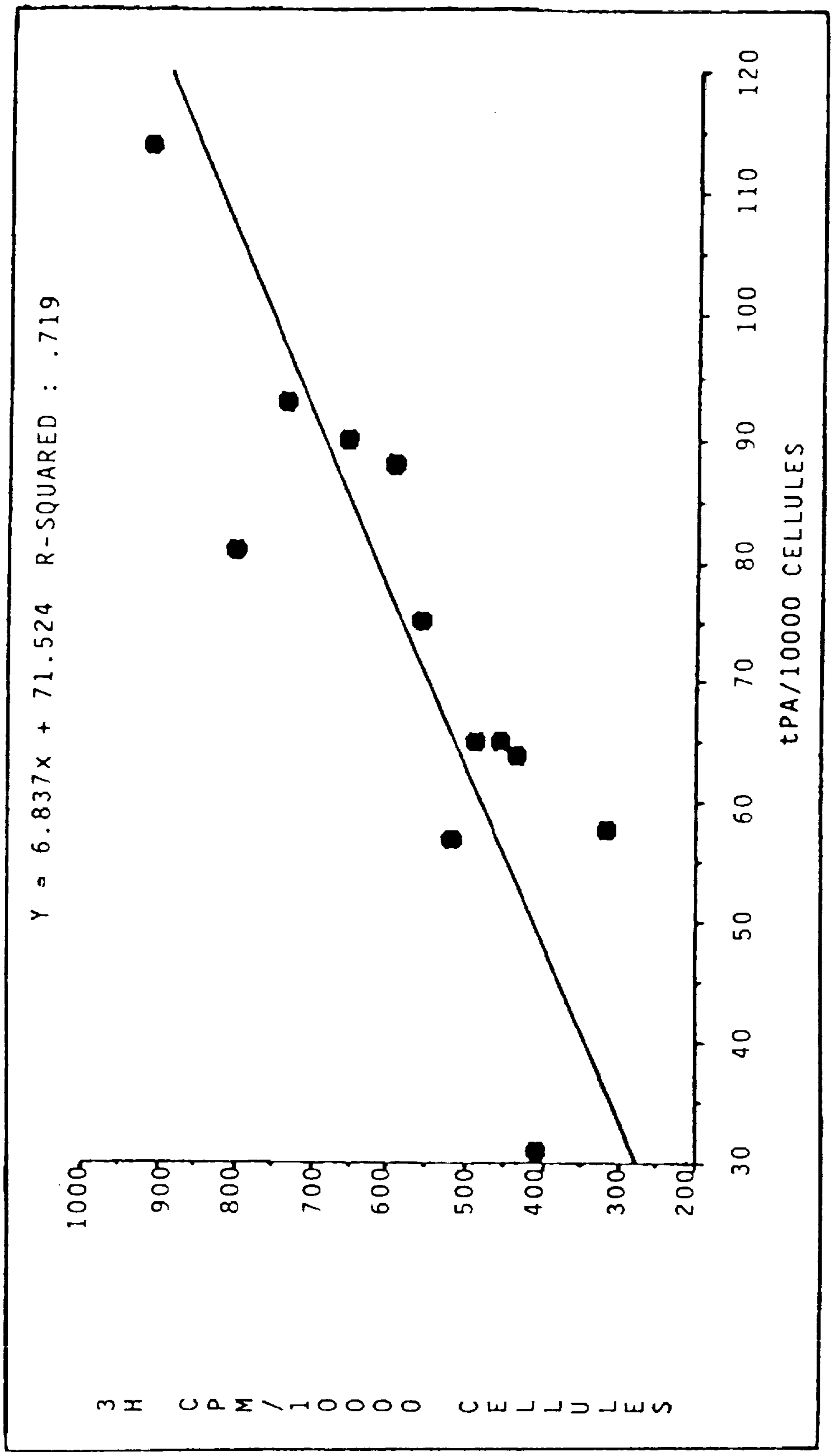
Y = 6.837x + 71.524 R-SQUARED : .719
1000
900
800
700
600
500
400
300
200
30
40
50
60
70
80
90
100
110
120
tPA/10000 CELLULES
3H CPM/10000 CELLULES

FIG. 2